# EUROPEAN PATENT SPECIFICATION

(11) **EP 0 640 098 B1**
(45) Date of publication and mention of the grant of the patent: **09.10.2002**
(21) Application number: 93911346.0
(22) Date of filing: 07.05.1993
(51) Int. Cl.: C07K 14/165, C12N 15/50, A61K 39/215, C12Q 1/70, C12Q 1/68

(54) **CANINE CORONAVIRUS S GENE AND USES THEREFOR**
HUNDECORONAVIRUS S GEN UND VERWENDUNG DAVON
GENE S DU CORONAVIRUS CANIN ET SES APPLICATIONS

(30) Priority: 08.05.1992 US 880194
(43) Date of publication of application: 01.03.1995
(73) Proprietor: PFIZER INC., New York, N.Y. 10017-5755 (US)
(72) Inventor: MILLER, Timothy, J., Malvern, PA 19355 (US); KLEPFER, Sharon, Broomall, PA 19008 (US); REED, Albert, Paul, Exton, PA 19341 (US); JONES, Elaine, V., Wynnewood, PA 19096 (US)
(74) Representative: Simpson, Alison Elizabeth Fraser
(86) International application number: US9304692
(87) International publication number: WO93023423

(56) References cited:
- EP-A- 0 264 979
- EP-A- 0 278 541
- EP-A- 0 376 744
- EP-A- 0 510 773
- EP-A- 0 524 672
- WO-A-93/23421
- WO-A-93/23422
- US-A- 4 904 468
- JOURNAL OF GENERAL VIROLOGY, vol. 73, 1992, GREAT BRITAIN, pages 2849-2862, XP002015843 B. C. HORSBURGH ET AL.,: "Analysis of a 9.6 kb sequence from the 3' end of canine coronavirus genomic RNA"
- VETERINARY MICROBIOLOGY, vol. 26, 1991, AMSTERDAM, NL, pages 25-40, XP000605182 G. F. RIMMELZWAAN: "The use of enzyme-linked immunosorbent assay systems for serology and antigen detection in parvovirus, coronavirus and rotavirus infections in dogs in The Netherlands "
- JOURNAL OF GENERAL VIROLOGY, vol. 75, 1994, GREAT BRITAIN, pages 1789-1794, XP002015276 J.G. WESSELING ET AL.: "Nucleotide sequence and expression of the spike (S) gene of canine coronavirus and comparison with the S proteins of feline and porcine coronaviruses"
- Proceedings of the National Academy of Sciences USA, Volume 80, issued March 1983, R.A. YOUNG et al., "Efficient Isolation of Genes by Using Antibody Probes", pp. 1194-1198, see entire document.
- Journal of General Virology, Volume 71, issued 1990, T. RAABE et al, "Nucleotide Sequence of the Gene Encoding the Spike Glycoprotein of Human Coronavirus HCV 229E", pp. 1065-1073, see Figure 4.
- Archives of Virology, Volume 117, issued 119, T. HOHDATSU et al., "Characterization of Monoclonal Antibodies Against Feline Infectious Peritonitis Virus Type II and Antigenic Relationship Between Feline, Porcine, and Canine Coronaviruses", pp. 85-95, see entire document.

## Description

### Field of the Invention

The present invention relates generally to canine coronavirus infections, and specifically to proteins useful in prophylaxis, therapy, and diagnosis of these infections in canines.

### Background of the Invention

The coronaviruses are a large family of mammalian and avian pathogens which were first described in 1968. They are the causative agents of several diseases including encephalitis, hepatitis, peritonitis and gastroenteritis. Enteric coronaviruses have been detected in the feces of man, pigs, calves, cats, mice, chickens and dogs.

Canine coronavirus (CCV) enteritis was first isolated from dogs suffering an acute gastroenteritis, as reported by Binn et al., Proc. 78th Ann. Mtg. U.S. Animal Health Assoc., Roanoke VA, pp. 359-366 (1974). The disease became prevalent during the 1970s. CCV gastroenteritis appears to be primarily transmitted through fecal contamination from infected dogs via the oral route, leading ultimately to replication of the virus in the epithelial cells of the small intestine. Virus can be recovered from the feces of an infected dog between 3 and 14 days after infection.

CCV gastroenteritis is characterized by a mild depression, anorexia and loose stool from which the dog usually recovers. The onset of the disease is often sudden, accompanied by such symptoms as diarrhea, vomiting, excreted blood in stools, and dehydration. Deaths have occurred within as little as 24 to 36 hours after onset of clinical signs. Most dogs appear afebrile but elevated body temperature is seen in some cases. Often CCV will occur with a canine parvovirus infection and this coinfection can be fatal.

Serologically the disease is closely related to transmissible gastroenteritis virus of swine (TGEV). Although canine coronavirus does not infect pigs, transmissible gastroenteritis virus produces a subclinical infection in dogs. However, unlike the feline infectious peritonitis coronavirus (FIPV), previous exposure to CCV does not predispose dogs to enhanced disease; and antigen-antibody complexes, if formed, are not associated with disease pathology.

A canine CCV vaccine comprising CCV peplomer protein is disclosed in US 4 904 468.

There remains a need in the art for compositions useful in diagnosing, treating and preventing infections with canine coronaviruses.

### Summary of the Invention

In one aspect the present invention provides the complete nucleotide sequence of the CCV S gene, strain 1-71, SEQ ID NO:1. The S gene may be useful in diagnostic compositions for CCV infection.

In another aspect the present invention provides a CCV S (or spike) protein characterized by the amino acid sequence of a CCV S protein, strain 1-71, SEQ ID NO:2. These proteins may be optionally fused or linked to other fusion proteins or molecules.

Thus, in another aspect, the present invention provides a vaccine composition containing an effective immunogenic amount of at least one CCV S protein, strain 1-71.

In still another aspect, the invention provides a method of vaccinating an animal against infection with a coronavirus by administering an effective amount of a vaccine composition of this invention.

In yet a further aspect, the present invention provides a pharmaceutical composition for the treatment of CCV infection comprising a therapeutically effective amount of a CCV S protein of the invention and a pharmaceutically effective carrier.

In yet another aspect, a diagnostic reagent of the present invention comprises a CCV S protein , strain 1-71. In another aspect, the present invention provides a diagnostic reagent which comprises a nucleotide sequence which encodes a CCV S protein, strain 1-71, and/or a nucleotide sequence which flanks the coding region. These protein and nucleotide sequences are optionally associated with detectable labels. Such diagnostic reagents may be used to assay for the presence of CCV in dogs using standard assay formats and can form components of a diagnostic kit.

In a further aspect, the invention provides a method of using a diagnostic reagent of this invention to identify dogs which are uninfected or which have been previously exposed to CCV. The diagnostic method can differentiate exposure to CCV from exposure to other related coronaviruses, allow the identification of dogs which have been vaccinated against these diseases, and allow one to distinguish between different strains of CCV, or to identify dogs at advanced stages of CCV infection.

In yet a further aspect, the invention provides a method for the production of a recombinant CCV S protein, strain 1-71, comprising culturing a selected host cell, e.g., a mammalian cell or viral vector, transformed with a DNA sequence encoding a selected CCV S protein, strain 1-71, in operative association with regulatory sequences capable of regulating the expression of said protein.

Other aspects and advantages of the present invention are described further in the following detailed description of the preferred embodiments thereof.

### Detailed Description of the Invention

The present invention provides novel isolated canine coronavirus (CCV) S proteins, strain 1-71, as well as isolated nucleotide sequences encoding the proteins. These proteins are useful for diagnostic, vaccinal and therapeutic compositions as well as methods for using these compositions in the diagnosis, prophylaxis and treatment of CCV-related and other coronavirus-related conditions.

### I. Definitions

As defined herein, an amino acid fragment is any amino acid sequence from at least about 8 amino acids in length up to about the full-length CCV S gene protein. A nucleotide fragment defines a nucleotide sequence which encodes from at least about 8 amino acids in length up to about the full-length CCV S gene protein.

The term "region" refers to all or a portion of a gene or protein, which may contain one or more fragments as defined above.

The term "immunogenic" refers to any S gene protein or fragment thereof, any molecule, protein, peptide, carbohydrate, virus, region or portion thereof which is capable of eliciting a protective immune response in a host, e.g., an animal, into which it is introduced.

The term "antigenic" refers only to the ability of a molecule, protein, peptide, carbohydrate, virus, region or portion thereof to elicit antibody formation in a host (not necessarily protective).

As used herein, the term "epitope" refers to a region of a protein which is involved in its immunogenicity, and can include regions which induce B cell and/or T cell responses.

As used herein, the term "B cell site or T cell site" defines a region of the protein which is a site for B cell or T cell binding. Preferably this term refers to sites which are involved in the immunogenicity of the protein.

### II. Sources of CCV Sequences

The examples below specifically refer to newly identified spike gene sequences from canine coronavirus (CCV) strain 1-71. This strain is deposited with the American Type Culture Collection (ATCC), 12301 Parklawn Drive, Rockville, Maryland under Accession No. VR-809. Particularly disclosed are nucleotide and amino acid sequences, SEQ ID NO:1 and 2, respectively, of the CCV S gene.

To facilitate an understanding of the operability of the invention, the description includes references to nucleotide fragments and peptide fragments of the CCV S protein and its nucleotide sequence. The nucleotide and peptide fragments are not part of the invention as claimed.

### III. CCV Nucleotide and Amino Acid Sequences of the Invention.

The inventors have identified and selected nucleotide and protein sequences of CCV strain 1-71 which have been determined to be of interest for use as vaccinal, therapeutic and/or diagnostic compositions. For example, selected peptide and nucleotide sequences present primarily in the variable N terminal region of the CCV S protein and gene are characterized by representing areas of homology between FIPV, TGEV, feline enteric coronavirus (FECV) and other coronavirus strains.

Peptide fragments obtained from this heterogeneous N terminal of the S protein are useful fragments for diagnostic compositions and kits for distinguishing between infection with CCV strain 1-71 from other CCV infections, and for distinguishing between infection with CCV and other coronavirus identified above in a vaccinated or infected dog, as well as for use in vaccine and therapeutic agents.

Additionally, the amino terminal sequences of CCV S protein include peptide sequences which are B cell sites and thus useful in vaccinal or therapeutic compositions, or for generating antibodies to CCV, in assays for the detection of CCV antibodies in dogs.

In addition, certain peptide fragments of the CCV S protein are believed to represent T cell sites, and thus are useful in vaccinal or therapeutic compositions.

Other suitable CCV amino acid regions for pharmaceutical or diagnostic use are located within other regions of the CCV S protein SEQ ID NO: 2. These amino acid and nucleotide fragments of the CCV S protein and its nucleotide sequence discussed above are specifically reported below in Tables I and II. Table II also reports the respective homologies of certain of these desired fragments to wild-type FIPV, i.e., FIPV WSU 1146. The CCV S nucleotide fragments in Tables I and II can be useful for diagnostic probes, PCR primers, or for use in recombinant production of relevant S protein fragments for use in therapeutic or vaccinal compositions. Other suitable fragments may also be identified for such use.

**Table I**

| CCV Amino Acids | | |
|---|---|---|
| B cell sites | T cell sites | SEQ ID NOS: |
| 50-250 | | 3 |
| 375-425 | | 4 |
| 450-470 | | 5 |
| 550-600 | | 6 |
| 650-700 | | 7 |
| 770-850 | | 8 |
| 900-1025 | | 9 |
| 1150-1225 | | 10 |
| 1250-1452 | | 11 |
| | 40-47 | 12 |
| | 63-81 | 13 |
| | 187-191 | 14 |
| | 241-274 | 15 |
| | 335-341 | 16 |
| | 395-428 | 17 |
| | 468-494 | 18 |
| | 846-860 | 19 |
| | 916-952 | 20 |
| | 977-992 | 21 |
| | 1068-1145 | 22 |
| | 1366-1391 | 23 |

**Table II**

| Amino Acid Sequences | | | |
|---|---|---|---|
| CCV 1-71 | | % Homology CCV 1-71 to WT FIPV WSU 1146 | SEQ ID NOS. AA Nucl. |
| Amino Acid | Nucleotides | | |
| 1113-1236 | 3337-3708 | 100 | 25 and 24 |
| 540-599 | 1618-1797 | 93.3 | 27 and 26 |
| 342-388 | 1024-1164 | 93.6 | 29 and 28 |
| 137-153 | 409-459 | 64.7 | 31 and 30 |
| 375-388 | 1123-1164 | 85.7 | 33 and 32 |
| 1424-1440 | 4270-4320 | 94.1 | 35 and 34 |
| 1407-1420 | 4219-4260 | 85.7 | 37 and 36 |
| 1342-1406 | 4024-4218 | 96.9 | 39 and 38 |
| 398-652 | 1192-1956 | 93.3 | 41 and 40 |
| 128-555 | 382-1665 | 89.5 | 43 and 42 |
| 447-628 | 1339-1884 | 91.8 | 45 and 44 |

### IV. Modified Sequences of the Invention.

In addition to the amino acid sequences and corresponding nucleotide sequences of the specifically-recited embodiments of CCV S proteins of this invention, allelic variations (naturally-occurring base changes in the species population which may or may not result in an amino acid change) of DNA sequences encoding the S protein are also included in the present invention. Similarly, DNA sequences which code for protein sequences of the invention but which differ in codon sequence due to the degeneracies of the genetic code or variations in the DNA sequence encoding these proteins which are caused by point mutations or by induced modifications to enhance the activity, half-life or production of the peptide encoded thereby are also encompassed in the invention.

Variations in the amino acid sequences of this invention may typically include analogs that differ by only 1 to about 4 codon changes. Other examples of analogs include proteins with minor amino acid variations from the natural amino acid sequence of S gene proteins and/or the fusion partner; in particular, conservative amino acid replacements. Conservative replacements are those that take place within a family of amino acids that are related in their side chains. Genetically encoded amino acids are generally divided into four families: (1) acidic = aspartate, glutamate; (2) basic = lysine, arginine, histidine; (3) non-polar = alanine, valine, leucine, isoleucine, proline, phenylalanine, methionine, tryptophan; and (4) uncharged polar = glycine, asparagine, glutamine, cysteine, serine, threonine, tyrosine. Phenylalanine, tryptophan, and tyrosine are sometimes classified jointly as aromatic amino acids. For example, it is reasonable to expect that an isolated replacement of a leucine with an isoleucine or valine, an aspartate with a glutamate, a threonine with a serine, or a similar conservative replacement of an amino acid with a structurally related amino acid will not have a significant effect on its activity, especially if the replacement does not involve an amino acid at an epitope of the polypeptides of this invention.

### V. Fusion Proteins.

If desired, the CCV S proteins and peptide fragments, e.g. those identified in Tables I and II, can be produced in the form of fusion proteins as defined below. Fusion proteins containing peptide fragments are not part of the invention as claimed. Such a fusion protein may contain either a full-length CCV S protein or an immunogenic fragment thereof. Suitable fragments include those contained within SEQ ID NO: 2 and the amino acids fragments of Tables I and II. Other suitable fragments can be determined by one of skill in the art by analogy to the sequences provided herein.

Proteins or peptides may be selected to form fusion proteins with the selected S protein or peptide sequence based on a number of considerations. The fusion partner may be a preferred signal sequence, a sequence which is characterized by enhanced secretion in a selected host cell system, or a sequence which enhances the stability or presentation of the S-derived peptide. Such exemplary fusion partners include, without limitation, ubiquitin and a mating factor for yeast expression systems, and beta-galactosidase and influenza NS-1 protein for bacterial systems. One of skill in the art can readily select an appropriate fusion partner for a selected expression system. The present invention is not limited to the use of any particular fusion partner.

The CCV S protein or fragments thereof can optionally be fused to each other or to the fusion partner through a conventional linker sequence, i.e., containing about 2 to 50 amino acids, and more preferably, about 2 to about 20 amino acids in length. This optional linker may provide space between the two linked sequences.
Alternatively, this linker sequence may encode, if desired, a polypeptide which is selectively cleavable or digestible by conventional chemical or enzymatic methods. For example, the selected cleavage site may be an enzymatic cleavage site, including sites for cleavage by a proteolytic enzyme, such as enterokinase, factor Xa, trypsin, collagenase and thrombin. Alternatively, the cleavage site in the linker may be a site capable of being cleaved upon exposure to a selected chemical, e.g., cyanogen bromide or hydroxylamine. The cleavage site, if inserted into a linker useful in the fused sequences of this invention, does not limit this invention. Any desired cleavage site, of which many are known in the art, may be used for this purpose.

### VI. Production of Sequences of Invention

The CCV S gene protein of the invention and amino acid regions, fragments thereof and their corresponding nucleotide sequences, as well as other proteins described herein, e.g. fusion partners, may be produced by conventional methods. These proteins or fragments and the nucleotide sequences may be prepared by chemical synthesis techniques [Merrifield, J.A.C.S., 85:2149-2154 (1963)]. Preferably, however, they are prepared by known recombinant DNA techniques by cloning and expressing within a host microorganism or cell a DNA fragment carrying a coding sequence for the selected protein. See, e.g., Sambrook et al, "Molecular Cloning. A Laboratory Manual", 2nd edit., Cold Spring Harbor Laboratory, New York (1989). Such techniques are discussed below in the Examples.

According to cloning techniques, a selected gene fragment can be cloned into a selected expression vector. Vectors for use in the method of producing S protein proteins comprise a novel S gene DNA sequence of the invention and selected regulatory sequences in operative association with the DNA coding sequence, and capable of directing the replication and expression of the peptide in a selected host cell.

Vectors, e.g., polynucleotide molecules, may be designed for expression of CCV S proteins and/or fusion proteins in bacterial, mammalian, fungal or insect cells or in selected viruses. Suitable vectors are known to one skilled in the art by resort to known publications or suppliers.

The resulting DNA molecules or vectors containing nucleotide sequences encoding the canine coronavirus S peptides or fragments thereof and/or encoding the fusion proteins are then introduced into host cells and expression of the heterologous protein induced.

Additional expression systems may include the known viral expression systems, e.g., vaccinia, fowlpox, swine pox. It is understood additionally, that the design of the expression vector will depend on the choice of host cell. A variety of suitable expression systems in any of the below-identified host cells are known to those skilled in the art and may be readily selected without undue effort.

Suitable cells or cell lines for use in expressing the S protein or peptides of this invention can be eukaryotic or prokaryotic. A preferred expression system includes mammalian cells, such as Chinese Hamster ovary cells (CHO) or COS-1 cells. The selection of other suitable mammalian host cells and methods for transformation, culture, amplification, screening and product production and purification are known in the art. See, e.g., Gething and Sambrook, Nature, 293:620-625 (1981), or alternatively, Kaufman et al, Mol. Cell. Biol., 5(7):1750-1759 (1985) or Howley et al, U. S. Patent 4,419,446. Also desirable are insect cell systems, such as the baculovirus or Drosophila systems. The selection of other suitable host cells and methods for transformation, culture, amplification, screening and product production and purification can be performed by one of skill in the art by reference to known techniques. See, e.g., Gething and Sambrook, Nature, 293:620-625 (1981).

After the transformed host cells are conventionally cultured for suitable times and under suitable culture conditions known to those skilled in the art, the cells may be lysed. It may also be possible, depending on the construct employed, that the recombinant proteins are secreted extracellularly and obtained from the culture medium. Cell lysates or culture medium are then screened for the presence of CCV S protein or peptide which are recognized by antibodies, preferably monoclonal antibodies (MAbs), to a peptide antigenic site from CCV.

Similarly, the fusion proteins may be produced by resort to chemical synthesis techniques, or preferably, recombinant methods, as described above. The selected primer sets used in the PCR reaction described in the Examples below may be designed to produce PCR amplified fragments containing restriction endonuclease cleavage site sequences for introduction of a canine coronavirus S gene fragment in a specific orientation into a selected expression vector to produce fusion proteins of the invention. The vector may contain a desired protein or fragment thereof to which the S gene fragment is fused in frame to produce a fusion protein.

The crude cell lysates containing the CCV S protein or peptides or fusion proteins can be used directly as vaccinal components, therapeutic compositions or diagnostic reagents. Alternatively, the CCV S peptides can be purified from the crude lysate or medium by conventional means.

### VII. Vaccine Compositions

The CCV S proteins of this invention may be incorporated in a vaccine composition. Such a vaccine composition may contain an immunogenic amount of one or more selected CCV S peptides or proteins, e.g., encoded by the complete S gene sequence of CCV or partial sequences thereof, and prepared according to the method of the present invention, together with a carrier suitable for administration as a vaccine composition for prophylactic treatment of CCV infections Vaccine compositions containing fragments or partial sequences of the complete S gene of CCV or fragments of the CCV S protein are not within the scope of the invention as claimed. The protein may be in the form of a fusion protein as above-described. Alternatively, the CCV S gene or fragment may be incorporated into a live vector, e.g., adenovirus, vaccinia virus and the like. The expression of vaccinal proteins in such live vectors are well-known to those in the art [See, e.g., U. S. Patent No. 4,920,209]. It is preferable that the protein employed in the vaccine composition induces protective immune responses against more than one strain of CCV.

A vaccine composition according to the invention may optionally contain other immunogenic components. Particularly desirable are vaccine compositions containing other canine antigens, e.g., canine distemper, *Borrelia burgdorferi*, canine *Bordetella*, rabies, canine parvovirus, *Leptosporidia* sp., canine rotavirus, canine parainfluenza virus and canine adenovirus.

In another embodiment, the CCV S proteins may be used in a combination vaccine directed to related coronaviruses. Other suitable coronaviruses which can be used in such a combination vaccine include a feline coronavirus, such as FIPV or FECV. For example, a CCV S peptide or protein of the present invention may be employed as an additional antigen in the temperature sensitive FIPV vaccine described in detail in Patent Application Ser. No. 07/428,796 (=US 5 667 785) filed October 30, 1989. Alternatively, the CCV S protein or peptide or a fragment thereof could be used in a vaccine composition containing other coronavirus S proteins or fragments thereof, particularly those described in co-pending, co-owned U.S. Patent application Ser. No. 07/698,927 (and its corresponding published PCT Application No. WO92/08487).

The preparation of a pharmaceutically acceptable vaccine composition, having appropriate pH isotonicity, stability and other conventional characteristics is within the skill of the art. Thus such vaccines may optimally contain other conventional components, such as adjuvants and/or carriers, e.g. aqueous suspensions of aluminum and magnesium hydroxides, liposomes and the like.

The vaccine composition may be employed to vaccinate animals against the clinical symptoms associated with CCV. The vaccines according to the present invention can be administered by an appropriate route, e.g., by the oral, intranasal, subcutaneous, intraperitoneal or intramuscular routes. The presently preferred methods of administration are the subcutaneous and intranasal routes.

The amount of the CCV S peptide or protein of the invention present in each vaccine dose is selected with regard to consideration of the animal's age, weight, sex, general physical condition and the like. The amount required to induce an immunoprotective response in the animal without significant adverse side effects may vary depending upon the recombinant protein employed as immunogen and the optional presence of an adjuvant.
Generally, it is expected that each dose will comprise between about 0.05-5000 micrograms of protein per mL, and preferably 0.05-100 micrograms per mL of a sterile solution of an immunogenic amount of a protein or peptide of this invention. Initial doses may be optionally followed by repeated boosts, where desirable.

Another vaccine agent of the present invention is an anti-sense RNA sequence generated to the S gene of CCV strain 1-71 [SEQ ID NO:1] [S. T. Crooke et al, Biotech., 10:882-886 (Aug. 1992)]. This sequence may easily be generated by one of skill in the art either synthetically or recombinantly. Under appropriate delivery, such an anti-sense RNA sequence when administered to an infected animal should be capable of binding to the RNA of the virus, thereby preventing viral replication in the cell.

### VIII. Pharmaceutical Compositions

The invention also provides a pharmaceutical composition comprising one or more CCV S peptides or proteins prepared according to the present invention and a pharmaceutically effective carrier. Suitable pharmaceutically effective carriers for internal administration are known to those skilled in the art. One selected carrier is sterile saline. The pharmaceutical composition can be adapted for administration by any appropriate route, but is designed preferentially for administration by injection or intranasal administration. *IX.*

The inventors also disclose the development of an antibody to one or more epitopes in the above identified amino acid sequences derived from the CCV S protein, which epitope is distinct from those of other CCV strains or other coronaviruses, e.g. FIPV, TGEV or FECV. The antibody can be developed employing as an antigenic substance, a peptide of Table I or II. Such an antibody is not part of the invention as claimed.
Alternatively, other regions of the CCV strain 1-71 S protein SEQ ID NO: 2 may be employed in the development of an antibody according to conventional techniques.

The antibody is capable of identifying or binding to a CCV antigenic site encoded by SEQ ID NO: 1 or a fragment thereof. Such an antibody may be used in a diagnostic screening test, e.g., as a hybridization probe, or as a therapeutic agent.

Antibodies which bind CCV peptides from the regions identified above or to other regions capable of distinguishing between CCV, TGEV, FIPV, FECV, and other coronaviruses for use in the assays may be polyclonal. However, it is desirable for purposes of increased target specificity to utilize MAbs, both in the assays and as potential therapeutic and prophylactic agents. Additionally, synthetically designed MAbs may be made by known genetic engineering techniques [W. D. Huse et al, Science, 246:1275-1281 (1989)] and employed in the methods described herein. For purposes of simplicity the term MAb(s) will be used throughout this specification; however, it should be understood that certain polyclonal antibodies, particularly high titer polyclonal antibodies and recombinant antibodies, may also be employed.

A MAb may be generated by the well-known Kohler and Milstein techniques and modifications thereof and directed to one or more of the amino acid residue regions identified above, or to other CCV S peptides or epitopes containing differences between CCV strain 1-71 and other coronaviruses. For example, a fragment of SEQ ID NO: 2 which represents an antigenic site, which differs from that of FIPV, may be presented as an antigen in conventional techniques for developing MAbs. One of skill in the art may generate any number of MAbs by using fragments of the amino acid residue regions identified herein as an immunogen and employing these teachings.

For diagnostic purposes, the antibodies (as well as the diagnostic probes) may be associated with individual labels. Where more than one antibody is employed in a diagnostic method, the labels are desirably interactive to produce a detectable signal. Most desirably, the label is detectable visually, e.g. colorimetrically. Detectable labels for attachment to antibodies useful in the diagnostic assays may also be easily selected by one skilled in the art of diagnostic assays, amont which include, without limitation, horseradish peroxidase (HRP) or alkaline phosphatase (AP), hexokinase in conjunction with glucose-6-phosphate dehydrogenase, and NAD oxidoreductase with luciferase and substrates NADH and FMN or peroxidase with luminol and substrate peroxide. These and other appropriate label systems and methods for coupling them to antibodies or peptides are known to those of skill in the art.

Antibodies may also be used therapeutically as targeting agents to deliver virus-toxic or infected cell-toxic agents to infected cells. Rather than being associated with labels for diagnostic uses, a therapeutic agent employs the antibody linked to an agent or ligand capable of disabling the replicating mechanism of the virus or of destroying the virally-infected cell. The identity of the toxic ligand does not limit the present invention. It is expected that preferred antibodies to peptides encoded by the S genes identified herein may be screened for the ability to internalize into the infected cell and deliver the ligand into the cell.

### X. Diagnostic Reagents and Assays

The nucleotide sequences, amino acid fragments and antibodies described above may be employed as diagnostic reagents for use in a variety of diagnostic methods.

### A. PCR Diagnostic Assays.

For example, these sequences can be utilized in a diagnostic method employing the polymerase chain reaction (PCR) technique to identify the presence of a CCV or CCV-like virus and in therapy of infected animals.

In addition to those sequences identified above, the oligonucleotide sequences that were designed to prime cDNA synthesis at specific sites within the CCV S gene, as described in detail below in Example 3 [SEQ ID NO:46-50], may also be employed as diagnostic reagents. These sequences, as well as the below-described optimized conditions for the PCR amplification of CCV fragments therefrom, may also be employed in a diagnostic method.

The PCR technique is known to those of skill in the art of genetic engineering and is described in detail in Example 4 [see, e.g., R. K. Saiki et al, Science, 230:1350-1354 (1985)], which is incorporated herein by reference. Briefly described, PCR employs two oligonucleotide primers which are complementary to the opposite strands of a double stranded nucleic acid of interest whose strands are oriented such that when they are extended by DNA polymerase, synthesis occurs across the region which separates the oligonucleotides. By repeated cycles of heat denaturation, annealing of the primers to their complementary sequences and extension of the annealed primers with a temperature stable DNA polymerase, millions of copies of the target gene sequence are generated. The template for the reaction is total RNA, which is isolated from CCV infected cells. DNA fragments generated by PCR were amplified from cDNA which had been synthesized from this RNA. Other strains of CCV or CCV-related sequences may also provide PCR templates in a similar manner.

In one diagnostic method, for example, heterogenous CCV gene sequences of this invention are useful as reagents in diagnostic assays to detect and distinguish the presence of specific viruses from each other, e.g., to distinguish one canine coronavirus strain from another or one species of coronavirus from another by means of conventional assay formats. For example, using protocols similar to those used for forensic purposes, tissue or blood samples from a dog suspected to be infected with CCV would be subjected to PCR amplification with a selected CCV-specific set of primers, such as those DNA sequences disclosed herein. Amplification of DNA from a sample tissue or biological fluid of the animal suspected of infection using nucleotide sequences as primers specific for regions of the CCV viral gene sequences could correlate to the presence of CCV. Absence of CCV in the sample would result in no amplification. Similarly, the selection of specific sets of S gene primers would allow the identification of a particular strain of CCV as well.. Thus, appropriate treatments may be selected for the infected animal.

Example 3 provides oligonucleotide primers which permitted the synthesis of regions of the CCV S gene. The nucleotide sequence of the S gene of CCV provides desirable sequences for hybridization probes and PCR primers, for example, the sequences between nucleotide base pairs 900 to about 1600 [SEQ ID NO: 55] and about 2500 to about 3900 [SEQ ID NO: 56] of SEQ ID NO: 1. Smaller or larger DNA fragments in these regions may also be employed as PCR primers or hybridization probes.

It is desirable to have PCR primer sequences between 15 to 30 bases in length, with an intervening sequence of at least 100 bases to as large as 5000 bases there between, according to conventional PCR technology. However, it is possible that larger or smaller sequence lengths may be useful based upon modifications to the PCR technology. In general, in order to achieve satisfactory discrimination, a hybridization or oligonucleotide probe made up of one or more of these sequences would consist of between 15 and 50 bases in length based on current technology.

### B. Conventional Assay Formats

The CCV S proteins or peptide fragments may also be employed in standard diagnostic assays which rely on S protein immunogens as targets for sera recognition. The diagnostic assays may be any conventionally employed assay, e.g., a sandwich ELISA assay, a Western blot, a Southern blot and the like. Because a wide variety of diagnostic methods exist and are conventionally known which can be adapted to the use of the nucleotide and amino acid sequences described herein, it should be understood that the nature of the diagnostic assay does not limit the use of the sequences disclosed in this application.

For example, the amino acid sequences encoded by CCV S gene sequences, such as those appearing in Tables I and II above, which may be amplified by PCR, provide peptides useful in such diagnostic assays as ELISA or Western assay, or as antigens for the screening of sera or development of antibodies.

For example, the sequences between about amino acid 1 to about 250 [SEQ ID NO:57), about 450 to about 650 [SEQ ID NO:58], and about 900 to about 1150 [SEQ ID NO:59] of the CCV strain 1-71 S gene protein SEQ ID NO:2, are anticipated to be useful as such antigens. Such peptides can optionally also be used in the design of synthetic peptide coupled to a carrier for diagnostic uses, e.g., antibody detection in sera. Suitable carriers include ovalbumin, keyhole limpet hemocyanin, bovine serum albumin, sepharose beads and polydextran beads.

Such peptide antigens and antibodies to these peptides would react positively with tissue or serum samples of dogs infected with CCV, but negatively with non-CCV infected dogs. These antibodies are discussed in more detail below.

For example, the invention provides a method of using the full length CCV S protein as diagnostic agents for identifying the presence or absence of antibodies in previously exposed, naive or vaccinated dogs, respectively, as well as for differentiating exposure to CCV from other related coronaviruses. Other S peptides or fusion proteins which show differential reactivity to CCV and other coronavirus sera may also be useful as CCV-specific reagents in ELISA-based screening assays to detect CCV exposure in dogs. Similarly, an S protein or peptide which contains epitopes recognized only by sera from CCV infected dogs or by sera from CCV positive dogs could be employed to distinguish or differentiate among coronavirus infections.

As one assay format, the reactivity of affinity purified CCV S proteins or peptides fragments to canine biological fluids or cells can be assayed by Western blot. The assay is preferably employed on sera, but may also be adapted to be performed on other appropriate fluids or cells, for example, macrophages or white blood cells. In the Western blot technique, the purified protein, separated by a preparative SDS polyacrylamide gel, is transferred to nitrocellulose and cut into multiple strips. The strips are then probed with dog sera from uninfected or infected dogs. Binding of the dog sera to the protein is detected by incubation with alkaline phosphatase tagged goat anti-dog IgG followed by the enzyme substrate BCIP/NBT. Color development is stopped by washing the strip in water.

CCV S protein or fragments thereof may also be used in an ELISA based assay for detecting CCV disease. A typical ELISA protocol would involve the adherence of antigen (e.g., a S protein) to the well of a 96-well tray. The serum to be tested is then added. If the serum contains antibody to the antigen, it will bind. Specificity of the reaction is determined by the antigen absorbed to the plate. With the S protein, only sera from those dogs infected with CCV would bind to the plate; sera from naive or uninfected dogs would not bind.

Similarly, a CCV S protein or peptide which contained epitopes recognized only by sera from CCV-infected dogs or by sera from CCV-positive dogs could be employed to distinguish coronavirus infections. After the primary antibody is bound, an enzyme-labeled antibody directed against the globulin of the animal whose serum is tested is added. Substrate is then added. The enzyme linked to antibody bound to the well will convert the substrate to a visible form. The amount of color measured is proportional to the amount of antibody in the test material. In this manner, dogs infected with CCV can be identified and treated, or dogs naive to the virus can be protected by vaccination.

When used as diagnostic reagents, the primers, probes, peptide antigens, nucleotide sequence encoding or flanking a CCV S protein of the invention, and antibodies disclosed in the application may be optionally associated with detectable labels or label systems known to those skilled in the art. Such labelled diagnostic reagents may be used to assay for the presence of CCV in dogs in hybridization assays or in the PCR technique as described above.

### C. Diagnostic Kits

The assay methods, PCR primers, CCV S nucleotide sequences [SEQ ID NO:1], S proteins and peptides, and antibodies described herein may be efficiently utilized in the assembly of a diagnostic kit, which may be used by veterinarians or laboratories. The kit is useful in distinguishing between CCV infected animals and vaccinated animals, as well as non-exposed dogs, and between CCV-infected animals and animals infected with serologically related viruses, such as other CCV or FIPV, TGEV, and FECV. Such a diagnostic kit contains the components necessary to practice the assays described above. Diagnostic kits containing antibodies are not part of the invention as claimed.

Thus, the kit may contain a sufficient amount of at least one CCV S protein, fusion protein or peptide fragment, at least one CCV S gene nucleotide sequence or PCR primer pair of this invention, a MAb directed to a first epitope on the CCV S protein (which MAb may be labeled), optional additional components of a detectable labelling system, vials for containing the serum samples, protein samples and the like, and a second MAb conjugated to the second enzyme, which in proximity to the first enzyme, produces a visible product. Other conventional components of such diagnostic kits may also be included.

Alternatively, a kit may contain a selected CCV S protein or peptide, a MAb directed against a selected CCV s peptide fragment bound to a solid surface and associated with a first enzyme, a different MAb associated with a second enzyme, and a sufficient amount of the substrate for the first enzyme, which, when added to the serum and MAbs, provides the reactant for the second enzyme, resulting in the color change.

Other known assay formats will indicate the inclusion of additional components for a diagnostic kit according to this invention.

The following examples illustrate the embodiments of this invention and do not limit the scope of the present invention.

### Example 1 - Isolation of CCV

Canine coronavirus strain 1-71 was isolated in 1971 from military dogs suffering from a viral gastroenteritis by Binn et al., Proceeding 78th Annual Meeting U.S. Animal Health Association, October 1974, p. 359-366. The initial isolate from the feces of the infected dog was grown in tissue culture on the PrDKTCA72 dog cell line [ATCC No. CRL 1542]. The coronavirus strain used in this study was received from the ATCC (ATCC #VR-809, CCV Strain 1-71, Frozen lot#4, Passage 7/PDK, 17 May 1988) and passaged five times on PrDKTCA72.

### Example 2 - RNA purification

After the fifth passage the infected cells were processed for RNA isolation by infecting a 1700 cc² roller bottle with a CCV inoculum. The inoculum was prepared by diluting 2.5 µl of infected fluids from a confluent monolayer into 13.0 mls of media. One ml of this material was used to infect a roller bottle and the cells were grown until they demonstrated a pronounced cytopathic effect at 48 hours. The infected monolayers were harvested and total cytoplasmic RNA was extracted using the guanidinium thiocyanate procedure as described in Chirgwin et al., Biochem., 18:5294 (1979).

### Example 3 - Primers Used for PCR Amplification of CCV Spike Gene Fragments

The primers appearing below in Table III were synthesized conventionally by the phosphoramidite method and gel purified prior to use. Primer #3045 was based on an FECV S gene sequence; and primers #4920, 1923, 2443 and 2600 were based on WT FIPV WSU 1146 sequences.

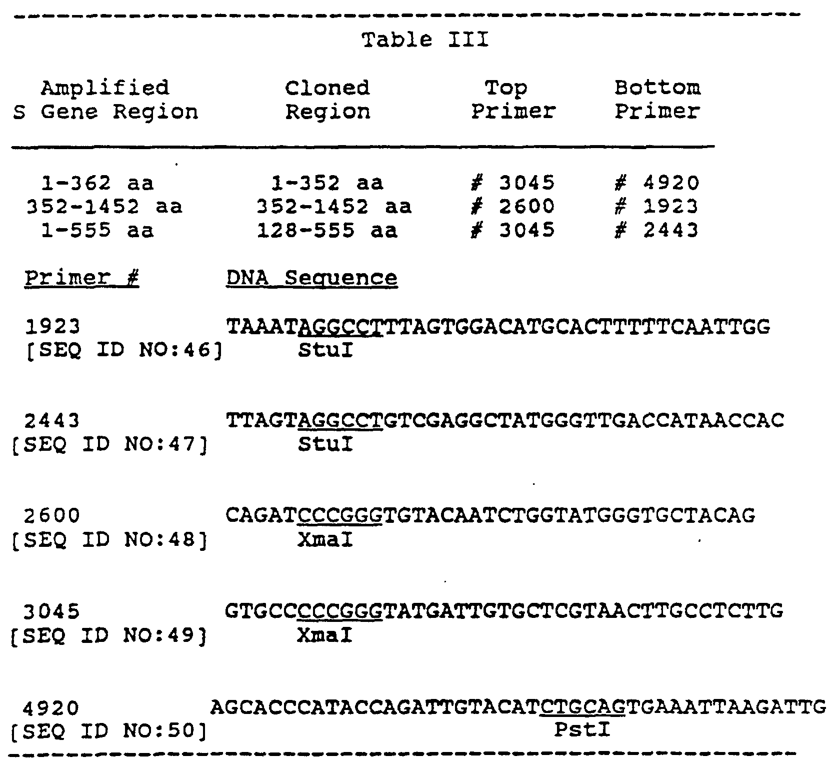

### Example 4 - PCR Amplification of CCV S Gene

PCR amplified fragments of CCV S gene were generated using the following procedure. All PCR reagents were supplied by Perkin Elmer-Cetus, Norwalk, CT. In a final reaction volume of 20 µl of 1X RT buffer (5X RT buffer: 250 mM Tris-HCl, pH 8.3, 375 mM KCl, 15 mM MgCl₂), the following components were assembled in RNAse-free siliconized 500 µl microcentrifuge tubes: 1.0 mM of each dNTP, 20 units of RNAsin [Promega Corp, Madison, WI], 2.5 picomoles of random hexamer oligonucleotides [Pharmacia, Milwaukee, WI], 100 picomoles/µl solution in TE buffer (10 mM Tris-HCl, 1 mM EDTA, pH 7.5), 200 units of reverse transcriptase [Superscript (RTM) RT, Bethesda Research Labs, Gaithersburg, MD] and 1.0 µg of respective RNA isolated as described above in Example 3. To avoid pipetting errors and contamination, all solutions were aliquoted from master mixes made with diethyl pyrocarbonate (DEPC) treated water and consisted of all of the reaction components except the RNA which was added last.

The mixture was incubated in a programmable thermal cycler [Perkin-Elmer Cetus, Norwalk, CT] at 21°C for ten minutes followed by 42°C for one hour then 95°C for five minutes and finally held at 4°C until PCR amplification.

Amplification of the cDNA was performed essentially according to the method of R. K. Saiki et al, Science, 230:1350-1354 (1985) using the Taq polymerase. Briefly, to the 20 µl cDNA reaction mix from above was added 10.0 µl 10X PCR buffer, 1.0 µl of each upstream and downstream primer previously diluted in water to 30 picomoles per microliter and 2.5 units of Taq polymerase (Perkin-Elmer Cetus, Norwalk, CT). Final volume was made up to 100 µl using DEPC treated water and overlaid with 100 µl of mineral oil. As above, master mixes were prepared to avoid contamination. The reaction was performed in the Perkin-Elmer Cetus thermal cycler for one cycle by denaturing at 95°C for 1 minute, annealing at 37°C for 3 minutes followed by an extension at 72°C for 40 minutes. This initial cycle increased the likelihood of first strand DNA synthesis. A standard PCR profile was then performed by a 95°C for 1 minute denaturation, 37°C for 3 minutes annealing, 72°C for 3 minutes extension for 40 cycles. A final extension cycle was done by 95°C for 1 minute denaturation, 37°C for 2 minutes annealing, 72°C for 15 minutes extension and held at 4°C until analyzed.

PCR products were analyzed by electrophoresing 5.0 µl of the reaction on a 1.2% agarose gel for 16-17 hours. Bands were visualized by ethidium bromide staining the gel and fluorescence by UV irradiation at 256 nm. Photography using Polaroid (RTM) type 55 film provided a negative that could be digitized for sample distance migration and comparison against markers run on each gel. The actual sizes of the bands were then calculated using the Beckman Microgenie software running on an IBM AT.

### Example 5 - Cloning of CCV Spike Gene Regions

Cloning procedures were performed substantially as described by Maniatis et al, cited above. Details of the clonings are provided in the following examples. Calf-alkaline phosphatase was from Bethesda Research Labs (Gaithersburg, MD). Ligation products were transformed into *E. coli* host strain XL1 Blue [Stratagene Cloning Systems, La Jolla, CA]. pBluescript SK_{Π}M13-phagemid vector was also obtained from Stratagene Cloning Systems. All restriction enzymes were purchased from New England Biolabs (Beverly, MA) or Bethesda Research Labs (Gaithersburg, MD) and used according to manufacturer's specifications. T4 DNA ligase was received from Boehringer Mannheim Biochemicals (Indianapolis, IN). Calf intestinal alkaline phosphatase was purchased from Bethesda Research Labs.

### Example 6 - CCV S Protein Fragment, A.A. 1-128 [SEQ ID NO:51] [Not covered by the scope of the claims]

Five microliters (approximately 200 ng) of PCR-amplified DNA representing amino acids 1-362 [SEQ ID NO:53] of the CCV spike gene were ligated to the pT7Blue T-Vector (Novagen, Madison, WI) as per the manufacturer's instructions. One microliter of the ligation mix was used to transform NovaBlue competent cells (Novagen) and transformation mixes were plated on LB plates supplemented with ampicillin, isopropylthio-β-galactoside (IPTG; Sigma Chemical Co., St. Louis, MO), and 5-bromo-4-chloro-3-indolyl-β-D-galactoside (X-gal; Sigma Chemical Co., St. Louis, MO). White colonies were picked and screened by restriction analysis of mini-prep DNA. Insert-bearing clones were identified and oriented with respect to vector by SmaI/PstI, StuI, and PstI digests. Clone #2964 contained a full-length 1-362 amino acid insert and was used to provide sequence analysis from 1-128 amino acids of the CCV S gene.

### Example 7 - CCV S Protein Fragment, A.A, 128-555 [SEQ ID NO:43] [Not covered by the scope of the claims]

10 µl of PCR DNA encoding 1-555aa of the CCV spike protein was digested with SmaI/StuI for 4 hours at room temperature. DNA bands were isolated and purified from low-melting temperature agarose gels as described by Maniatis et al, cited above. Briefly, DNA fragments were visualized after staining with ethidium bromide, excised from the gel with a scalpel and transferred to microfuge tubes. Gel slices were incubated 5 min at 65°C, vortexed, and 5 volumes of 20 mM Tris, pH 8.0, 1 mM EDTA were added. Samples were incubated an additional 2 minutes at 65°C and were then extracted once with phenol and again with phenol:chloroform. The DNA was precipitated with 1/10 volume 3 M NaOAc, pH 7.0, and 2.5 volumes of cold 95% EtOH overnight at -20°C. Insert DNAs were ligated to SK_{Π}M13-SmaI-digested, dephosphorylated vector [Stratagene] for 4 hours at room temperature. Insert-bearing clones were identified by XhoI/SstI and BglI digests of mini-prep DNA. Restriction enzyme and sequence analysis indicated that the cloned insert was short by ⁻300bp due to the presence of a StuI site at amino acid #128 of the CCV spike gene. Therefore, these clones contained the CCV S protein spanning amino acids from about 128-555 [SEQ ID NO:43].

### Example 8 - CCV S Protein Fragment. A.A. 352-1452 [SEQ ID NO:52] [Not covered by the scope of the claims]

PCR-amplified DNA fragments encoding amino acids 352-1454 of the CCV spike protein were purified using Prime-Erase Quik Columns [Stratagene] according to the manufacturer's instructions. Column-purified DNAs were then digested with XmaI/EcoRV overnight at 15°C and subsequently isolated and eluted from low-melting temperature agarose gels as described by Maniatis et al, cited above. Inserts were ligated overnight at 15°C to SK_{Π}M13- XmaI/StuI digested, dephosphorylated vector [Stratagene]. Clones were identified and oriented with respect to vector by XhoI/SstI and PvuII digests of mini-prep DNAs, respectively.

### Example 9 - DNA Sequencing

DNA sequence for the CCV S gene was determined from the individual clones #1775 (AA 352-1452; SEQ ID NO:52), #2007 (AA 128-555; SEQ ID NO:43) and #2964 (AA 1-362; SEQ ID NO:53). Nested set deletions were prepared from each clone or internal primers synthesized to facilitate primer walking and the sequence determined from both strands [Lark Sequencing Technologies, Houston, TX]. The chain termination method performed as described in Sanger et al, Proc. Natl. Acad. Sci. USA, 74:5463-5467 (1977) was used to determine the sequence of all clones. The full length sequence of the CCV S gene was assembled from overlapping sequences of each of the three separate fragments by computer analysis.

DNA sequence analysis was performed using either Beckman Microgenie programs on an IBM Model PS/2 Model 70 or the University of Wisconsin GCG package of programs implemented on a DEC VAX cluster [Devereau et al., (1984)].

SEQ ID NO:1 is the complete nucleotide sequence of the CCV strain 1-71 S gene. The amino acid [SEQ ID NO:2] and nucleotide sequences [SEQ ID NO:1] of CCV 1-71 total 1452 amino acids and 4356 base pairs. CCV 1-71 has a DNA homology of 90.8% to published FIPV strain WT WSU 1146, 93.2% identity with FIPV strain DF2 and 94.1% similarity with FECV. In comparison to WSU 1146, this CCV strain further contains two amino acid deletions at positions 11 and 12, and two amino acid insertions at positions 118 and 119. In comparison to the amino acid sequences of other coronavirus S genes, the amino acid sequence of CCV is 82.2% homologous to TGEV, 89.7% homologous to DF2-HP, 90.0% homologous to TS-BP, 92.9% homologous to TS, 93.2% homologous to DF2, and 94.1% homologous to FECV.

The canine coronavirus S gene encoding amino acids #225-1325 [SEQ ID NO:54] has an overall homology to the published WT FIPV WSU 1146 strain at amino acids 352 to 1454 of 95.9%. The homology level is increased to 97.5% when the comparison is done under the amino acid similarity rules as proposed by M. O. Dayhoff, Atlas of Protein Sequence and Structure, Vol. 5, Supp. 3, Natl. Biomed. Res. Found., Washington, DC (1978). There are 42 amino acid differences between the CCV S gene and the published sequence of WSU 1146 strain within the CCV sequence of SEQ ID NO: 2. other CCV fragment homologies with WT FIPV WSU 1146 are illustrated in Table II above.

## Claims

1. An isolated protein sequence comprising amino acid residues 1 to 1452 of the S protein of canine coronavirus strain CCV 1-71 (SEQ ID NO: 2).

2. A protein sequence according to Claim 1 wherein the protein is fused to a selected protein.

3. A DNA sequence encoding the S protein of canine coronavirus strain CCV 1-71 selected from SEQ ID NO: 1 or a sequence that encodes SEQ ID NO: 2.

4. A DNA sequence according to Claim 3 further comprising a DNA sequence encoding a selected protein capable of being fused to the S protein of canine coronavirus strain CCV 1-71.

5. A method for the production of recombinant canine coronavirus strain CCV 1-71 S protein comprising culturing a selected host transformed with a DNA sequence according to Claim 3 in operative association with one or more regulatory sequences capable of regulating expression of said protein.

6. A method according to Claim 5 wherein said host is a mammalian cell.

7. A method according to Claim 5 wherein said host is a viral vector.

8. A recombinant vector comprising the DNA sequence according to Claim 3 said sequence in operative association with one or more regulatory elements capable of directing the expression thereof.

9. A host cell comprising the recombinant vector of Claim 8.

10. A vaccine composition comprising an effective amount of the S protein of canine coronavirus strain CCV 1-71 and an optional carrier.

11. A vaccine composition according to Claim 10 wherein said S protein is a fusion protein.

12. A vaccine composition according to Claims 10 or 11 further comprising an immunogenic amount of one or more additional antigens.

13. Use of a canine coronavirus protein comprising amino acid residues 1 to 1452 of the S protein of canine coronavirus strain CCV 1-71 optionally fused to a selected protein, for the manufacture of a medicament for vaccination against, or treatment of, canine coronavirus infections.

14. A diagnostic kit comprising one or more diagnostic reagents selected from the group consisting of:
a) a full length sequence of the S protein of canine coronavirus strain CCV 1-71 comprising amino acid residues 1 to 1452, optionally fused to a selected protein, said sequence optionally associated with a detectable label: and
b) a nucleotide sequence encoding canine coronavirus strain CCV 1-71 S protein, said nucleotide sequence optionally associated with a detectable label.

15. Use of a kit according to Claim 14 for the in vitro diagnosis of CCV infection in dogs.

16. Use of a kit according to Claim 15 wherein dogs previously exposed to CCV or to a CCV vaccine arc detected.

17. Use of a kit according to Claim 15 wherein said in vitro diagnosis can differentiate exposure to canine coronavirus strain CCV 1-71 from exposure to other related coronavirus.

## Patentansprüche

1. Isolierte Proteinsequenz mit den Aminosäureresten 1 bis 1452 des S-Proteins des Coronavirusstamms CCV 1-71 vom Hund (SEQ ID Nr. 2).

2. Proteinsequenz nach Anspruch 1, wobei das Protein mit einem ausgewählten Protein fusioniert ist.

3. DNA-Sequenz, die das S-Protein von Hundecoronavirusstamm CCV 1-71 codiert ausgewählt aus SEQ ID Nr. 1 oder einer Sequenz, die SEQ ID Nr. 2 codiert.

4. DNA-Sequenz nach Anspruch 3, die weiterhin eine DNA-Sequenz umfasst, die ein ausgewähltes Protein codiert, das mit dem S-Protein von Hundecoronavirusstamm CCV 1-71 fusioniert werden kann.

5. Verfahren zur Herstellung eines rekombinanten S-Proteins des Hundecoronavirusstamms CCV 1-71 umfassend, dass ein ausgewählter Wirt gezüchtet wird, der mit einer DNA-Sequenz gemäß Anspruch 3 in operativer Verbindung mit einer oder mehreren regulatorischen Sequenzen, die die Expression dieses Proteins regulieren können, transformiert ist.

6. Verfahren nach Anspruch 5, wobei der Wirt eine Säugetierzelle ist.

7. Verfahren nach Anspruch 5, wobei der Wirt ein viraler Vektor ist.

8. Rekombinanter Vektor umfassend die DNA-Sequenz nach Anspruch 3, wobei die Sequenz operativ verbunden ist mit einer oder mehreren regulatorischen Elementen, die dessen Expression regulieren können.

9. Wirtszelle umfassend den rekombinanten Vektor nach Anspruch 8.

10. Impfstoffzusammensetzung umfassend eine wirksame Menge des S-Proteins von Hundecoronavirusstamm CCV 1-71 und einen fakultativen Träger.

11. Impfstoffzusammensetzung nach Anspruch 10, wobei das S-Protein ein Fusionsprotein ist.

12. Impfstoffzusammensetzung nach Anspruch 10 oder Anspruch 11 weiter umfassend eine immunogene Menge eines oder mehrerer zusätzlicher Antigene.

13. Verwendung eines Hundecoronavirusproteins umfassend die Aminosäurereste 1 bis 1452 des S-Proteins von Hundecoronavirusstamm CCV 1-71, gegebenenfalls fusioniert mit einem ausgewählten Protein zur Herstellung eines Arzneimittels zur Impfung gegen Hundecoronavirusinfektionen oder zu deren Behandlung.

14. Diagnosekit umfassend ein oder mehrere diagnostische Reagenzien ausgewählt aus der Gruppe bestehend aus:
a) einer Sequenz des S-Proteins von Hundecoronavirusstamm CCV 1-71 mit den Aminosäureresten 1 bis 1452 in voller Länge, gegebenenfalls fusioniert mit einem ausgewählten Protein, wobei die Sequenz gegebenenfalls mit einer nachweisbaren Markierung verbunden ist und
b) einer Nucleotidsequenz, die S-Protein von Hundecoronavirusstamm CCV 1-71 codiert, wobei die Nucleotidsequenz gegebenenfalls mit einer nachweisbaren Markierung verbunden ist.

15. Verwendung eines Kits nach Anspruch 14 zur in-vitro-Diagnose von CCV-Infektionen bei Hunden.

16. Verwendung eines Kits nach Anspruch 15, mit dem bei Hunden nachgewiesen wird, ob sie vorher CCV oder einem CCV-Impfstoff ausgesetzt waren.

17. Verwendung eines Kits nach Anspruch 15, wobei die invitro-Diagnose zwischen dem Kontakt mit einem Hundecoronavirusstamm CCV 1-71 und einem anderen verwandten Coronavirus differenzieren kann.

## Revendications

1. Séquence de protéine isolée comprenant les résidus d'amino-acides 1 à 1452 de la protéine S de la souche de coronavirus canin CCV 1-71 (SEQ ID N° 2).

2. Séquence de protéine suivant la revendication 1, dans laquelle la protéine est fusionnée à une protéine choisie.

3. Séquence d'ADN codant pour la protéine S de la souche de coronavirus canin CCV 1-71 choisie entre la SEQ ID N° 1 et une séquence qui code pour la SEQ ID N° 2.

4. Séquence d'ADN suivant la revendication 3, comprenant en outre une séquence d'ADN codant pour une protéine choisie pouvant être fusionnée à la protéine S de la souche de coronavirus canin CCV 1-71.

5. Procédé pour la production d'une protéine S recombinante de la souche de coronavirus canin CCV 1-71, comprenant la culture d'un hôte choisi transformé avec une séquence d'ADN suivant la revendication 3 en association fonctionnelle avec une ou plusieurs séquences régulatrices capables de réguler l'expression de ladite protéine.

6. Procédé suivant la revendication 5, dans lequel l'hôte est une cellule de mammifère.

7. Procédé suivant la revendication 5, dans lequel l'hôte est un vecteur viral.

8. Vecteur recombinant comprenant la séquence d'ADN suivant la revendication 3, ladite séquence étant en association fonctionnelle avec un ou plusieurs éléments régulateurs capables de diriger l'expression de cette séquence d'ADN.

9. Cellule hôte comprenant le vecteur recombinant suivant la revendication 8.

10. Composition de vaccin comprenant une quantité efficace de la protéine S de la souche de coronavirus canin CCV 1-71 et un support facultatif.

11. Composition de vaccin suivant la revendication 10, dans laquelle la protéine S est une protéine de fusion.

12. Composition de vaccin suivant la revendication 10 ou 11, comprenant une quantité immunogène d'un ou plusieurs antigènes supplémentaires.

13. Utilisation d'une protéine de coronavirus canin, comprenant les résidus d'amino-acides 1 à 1452 de la protéine S de la souche de coronavirus canin CCV 1-71, facultativement fusionnée à une protéine choisie, pour la production d'un médicament destiné à vacciner contre, ou traiter, des infections par des coronavirus canins.

14. Kit de diagnostic comprenant un ou plusieurs réactifs de diagnostic choisis dans le groupe consistant en :
a) une séquence totale de la protéine S de la souche de coronavirus canin CCV 1-71 comprenant les résidus d'amino-acides 1 à 1452, facultativement fusionnée à une protéine choisie, ladite séquence étant facultativement associée à un marqueur détectable ; et
b) une séquence de nucléotides codant pour la protéine S de la souche de coronavirus canin CCV 1-71, ladite séquence de nucléotides étant facultativement associée à un marqueur détectable.

15. Utilisation d'un kit suivant la revendication 14 pour le diagnostic in vitro d'une infection par le CCV chez le chien.

16. Utilisation d'un kit suivant la revendication 15, dans laquelle des chiens préalablement exposés au CCV ou à un vaccin contre le CCV sont détectés.

17. Utilisation d'un kit suivant la revendication 15, dans laquelle le diagnostic in vitro permet de faire la différence entre l'exposition à la souche de coronavirus canin CCV 1-71 et l'exposition à d'autres coronavirus apparentés.


### SEQUENCE LISTING

(1) GENERAL INFORMATION:
   (i) APPLICANT: Miller, Timothy J.
      Klepfer, Sharon
      Reed, Albert Paul
      Jones, Elaine V.
   (ii) TITLE OF INVENTION: Canine Coronavirus S Gene and Uses Therefor
   (iii) NUMBER OF SEQUENCES: 59
   (iv) CORRESPONDENCE ADDRESS:
      (A) ADDRESSEE: SmithKline Beecham Corporation - Corporate Patents
      (B) STREET: 709 Swedeland Road
      (C) CITY: King of Prussia
      (D) STATE: PA
      (E) COUNTRY: USA
      (F) ZIP: 19406-2799
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US
      (B) FILING DATE:
      (C) CLASSIFICATION:
   (vii) PRIOR APPLICATION DATA:
      (A) APPLICATION NUMBER: US 07/880,194
      (B) FILING DATE: 08-MAY-1992
   (vii) PRIOR APPLICATION DATA:
      (A) APPLICATION NUMBER: US 07/698,927
      (B) FILING DATE: 13-MAY-1991
   (vii) PRIOR APPLICATION DATA:
      (A) APPLICATION NUMBER: US 07/613,066
      (B) FILING DATE: 14-NOV-1990
   (viii) ATTORNEY/AGENT INFORMATION:
      (A) NAME: Schreck, Patrica A.
      (B) REGISTRATION NUMBER: 33,777
      (C) REFERENCE/DOCKET NUMBER: SBC H85010-1
   (ix) TELECOMMUNICATION INFORMATION:
      (A) TELEPHONE: (215) 270-5015
      (B) TELEFAX: (215) 270-5090
(2) INFORMATION FOR SEQ ID NO:1:
   (i) SEQUENCE CHARACTERISTICS:
      (A) LENGTH: 4359 base pairs
      (B) TYPE: nucleic acid
      (C) STRANDEDNESS: double
      (D) TOPOLOGY: unknown
   (ii) MOLECULE TYPE: DNA (genomic)
   (ix) FEATURE:
      (A) NAME/KEY: CDS
      (B) LOCATION: 1..4356
   (xi) SEQUENCE DESCRIPTION: SEQ ID NO:1:
   ATG ATT GTG CTC GTA ACT TGC CTC TTG TTT TCG TAC AAT AGT GTG ATT        48
   Met Ile Val Leu Val Thr Cys Leu Leu Phe Ser Tyr Asn Ser Val Ile
     1               5                  10                  15
   TGT ACA TCA AAC AAT GAC TGT GTA CAA GTT AAT GTG ACA CAA TTG CCT        96
   Cys Thr Ser Asn Asn Asp Cys Val Gln Val Asn Val Thr Gln Leu Pro
                20                  25                  30
   GGC AAT GAA AAC ATT ATT AAA GAT TTT CTA TTT CAC ACC TTC AAA GAA       144
   Gly Asn Glu Asn Ile Ile Lys Asp Phe Leu Phe His Thr Phe Lys Glu
            35                  40                  45
   GAA GGA AGT GTA GTT GTT GGT GGT TAT TAC CCT ACA GAG GTG TGG TAT       192
   Glu Gly Ser Val Val Val Gly Gly Tyr Tyr Pro Thr Glu Val Trp Tyr
        50                  55                  60
   AAC TGC TCC AGA AGC GCA ACA ACC ACC GCT TAC AAG GAT TTT AGT AAT       240
   Asn Cys Ser Arg Ser Ala Thr Thr Thr Ala Tyr Lys Asp Phe Ser Asn
    65                  70                  75                  80
   ATA CAT GCA TTC TAT TTT GAT ATG GAA GCC ATG GAG AAT AGT ACT GGC       288
   Ile His Ala Phe Tyr Phe Asp Met Glu Ala Met Glu Asn Ser Thr Gly
                    85                  90                  95
   AAT GCA CGA GGT AAA CCT TTA CTA GTA CAT GTT CAT GGT GAT CCT GTT       336
   Asn Ala Arg Gly Lys Pro Leu Leu Val His Val His Gly Asp Pro Val
               100                 105                 110
   AGT ATC ATC ATA TAT ATA TCG GCT TAT AGA GAT GAT GTG CAA GGA AGG       384
   Ser Ile Ile Ile Tyr Ile Ser Ala Tyr Arg Asp Asp Val Gln Gly Arg
           115                 120                 125
   CCT CTT TTA AAA CAT GGT TTG TTG TGT ATA ACT AAA AAT AAA ATC ATT       432
   Pro Leu Leu Lys His Gly Leu Leu Cys Ile Thr Lys Asn Lys Ile Ile
       130                 135                 140
   GAC TAT AAC ACG TTT ACC AGC GCA CAG TGG AGT GCC ATA TGT TTG GGT       480
   Asp Tyr Asn Thr Phe Thr Ser Ala Gln Trp Ser Ala Ile Cys Leu Gly
   145                 150                 155                 160
   GAT GAC AGA AAA ATA CCA TTC TCT GTC ATA CCC ACA GGT AAT GGT ACA       528
   Asp Asp Arg Lys Ile Pro Phe Ser Val Ile Pro Thr Gly Asn Gly Thr
                   165                 170                 175
   AAA ATA TTT GGT CTT GAG TGG AAT GAT GAC TAT GTT ACA GCC TAT ATT       576
   Lys Ile Phe Gly Leu Glu Trp Asn Asp Asp Tyr Val Thr Ala Tyr Ile
               180                 185                 190
   AGT GAT CGT TCT CAC CAT TTG AAC ATC AAT AAT AAT TGG TTT AAC AAT       624
   Ser Asp Arg Ser His His Leu Asn Ile Asn Asn Asn Trp Phe Asn Asn
           195                 200                 205
   GTG ACA ATC CTA TAC TCT CGA TCA AGC ACT GCT ACG TGG CAG AAG AGT       672
   Val Thr Ile Leu Tyr Ser Arg Ser Ser Thr Ala Thr Trp Gln Lys Ser
       210                 215                 220
   GCT GCA TAT GTT TAT CAA GGT GTT TCA AAT TTT ACT TAT TAC AAG TTA       720
   Ala Ala Tyr Val Tyr Gln Gly Val Ser Asn Phe Thr Tyr Tyr Lys Leu
   225                 230                 235                 240
   AAT AAC ACC AAT GGC TTG AAA AGC TAT GAA TTG TGT GAA GAT TAT GAA       768
   Asn Asn Thr Asn Gly Leu Lys Ser Tyr Glu Leu Cys Glu Asp Tyr Glu
                   245                 250                 255
   TGC TGC ACT GGC TAT GCT ACC AAC GTA TTT GCC CCG ACA GTG GGC GGT       816
   Cys Cys Thr Gly Tyr Ala Thr Asn Val Phe Ala Pro Thr Val Gly Gly
               260                 265                 270
   TAT ATA CCT GAT GGC TTC AGT TTT AAC AAT TGG TTT ATG CTT ACA AAC       864
   Tyr Ile Pro Asp Gly Phe Ser Phe Asn Asn Trp Phe Met Leu Thr Asn
           275                 280                 285
   AGT TCC ACG TTT GTT AGT GGC AGA TTT GTA ACA AAT CAA CCA TTA TTG       912
   Ser Ser Thr Phe Val Ser Gly Arg Phe Val Thr Asn Gln Pro Leu Leu
       290                 295                 300
   GTT AAT TGT TTG TGG CCA GTG CCC AGT CTT GGT GTC GCA GCA CAA GAA       960
   Val Asn Cys Leu Trp Pro Val Pro Ser Leu Gly Val Ala Ala Gln Glu
   305                 310                 315                 320
   TTT TGT TTT GAA GGT GCG CAG TTT AGC CAA TGT AAT GGT GTG TCT TTA      1008
   Phe Cys Phe Glu Gly Ala Gln Phe Ser Gln Cys Asn Gly Val Ser Leu
                   325                 330                 335
   AAC AAT ACA GTG GAT GTC ATT AGA TTC AAC CTT AAT TTT ACC ACA GAT      1056
   Asn Asn Thr Val Asp Val Ile Arg Phe Asn Leu Asn Phe Thr Thr Asp
               340                 345                 350
   GTA CAA TCT GGT ATG GGT GCT ACA GTA TTT TCA CTG AAT ACA ACA GGT      1104
   Val Gln Ser Gly Met Gly Ala Thr Val Phe Ser Leu Asn Thr Thr Gly
           355                 360                 365
   GGT GTC ATT CTT GAG ATT TCT TGT TAT AAT GAT ACA GTG AGT GAG TCA      1152
   Gly Val Ile Leu Glu Ile Ser Cys Tyr Asn Asp Thr Val Ser Glu Ser
       370                 375                 380
   AGT TTC TAC AGT TAT GGT GAA ATT TCA TTC GGC GTA ACT GAT GGA CCG      1200
   Ser Phe Tyr Ser Tyr Gly Glu Ile Ser Phe Gly Val Thr Asp Gly Pro
   385                 390                 395                 400
   CGT TAC TGT TAC GCA CTC TAT AAT GGC ACG GCT CTT AAG TAT TTA GGA      1248
   Arg Tyr Cys Tyr Ala Leu Tyr Asn Gly Thr Ala Leu Lys Tyr Leu Gly
                   405                 410                 415
   ACA TTA CCA CCT AGT GTC AAG GAA ATT GCT ATT AGT AAG TGG GGC CAT      1296
   Thr Leu Pro Pro Ser Val Lys Glu Ile Ala Ile Ser Lys Trp Gly His
               420                 425                 430
   TTT TAT ATT AAT GGT TAC AAT TTC TTT AGC ACT TTT CCT ATT GAT TGT      1344
   Phe Tyr Ile Asn Gly Tyr Asn Phe Phe Ser Thr Phe Pro Ile Asp Cys
           435                 440                 445
   ATA TCT TTT AAT TTA ACC ACT GGT GAT AGT GGA GCA TTT TGG ACA ATT      1392
   Ile Ser Phe Asn Leu Thr Thr Gly Asp Ser Gly Ala Phe Trp Thr Ile
       450                 455                 460
   GCT TAC ACA TCG TAC ACT GAC GCA TTA GTA CAA GTT GAA AAC ACA GCT      1440
   Ala Tyr Thr Ser Tyr Thr Asp Ala Leu Val Gln Val Glu Asn Thr Ala
   465                 470                 475                 480
   ATT AAA AAG GTG ACG TAT TGT AAC AGT CAC ATT AAT AAC ATT AAA TGT      1488
   Ile Lys Lys Val Thr Tyr Cys Asn Ser His Ile Asn Asn Ile Lys Cys
                   485                 490                 495
   TCT CAA CTT ACT GCT AAT TTG CAA AAT GGA TTT TAT CCT GTT GCT TCA      1536
   Ser Gln Leu Thr Ala Asn Leu Gln Asn Gly Phe Tyr Pro Val Ala Ser
               500                 505                 510
   AGT GAA GTT GGT CTT GTC AAT AAG AGT GTT GTG TTA CTA CCT AGT TTC      1584
   Ser Glu Val Gly Leu Val Asn Lys Ser Val Val Leu Leu Pro Ser Phe
           515                 520                 525
   TAT TCA CAT ACC AGT GTT AAT ATA ACT ATT GAT CTT GGT ATG AAG CGT      1632
   Tyr Ser His Thr Ser Val Asn Ile Thr Ile Asp Leu Gly Met Lys Arg
       530                 535                 540
   AGT GGT TAT GGT CAA CCC ATA GCC TCA ACA TTA AGT AAC ATC ACA CTA      1680
   Ser Gly Tyr Gly Gln Pro Ile Ala Ser Thr Leu Ser Asn Ile Thr Leu
   545                 550                 555                 560
   CCA ATG CAG GAT AAT AAC ACC GAT GTG TAC TGC ATT CGT TCT AAC CAA      1728
   Pro Met Gln Asp Asn Asn Thr Asp Val Tyr Cys Ile Arg Ser Asn Gln
                   565                 570                 575
   TTT TCA GTT TAC GTT CAT TCC ACT TGT AAA AGT TCT TTA TGG GAC GAT      1776
   Phe Ser Val Tyr Val His Ser Thr Cys Lys Ser Ser Leu Trp Asp Asp
               580                 585                 590
   GTG TTT AAT TCC GAC TGC ACA GAT GTT TTA TAT GCT ACA GCT GTT ATA      1824
   Val Phe Asn Ser Asp Cys Thr Asp Val Leu Tyr Ala Thr Ala Val Ile
           595                 600                 605
   AAA ACT GGT ACT TGT CCT TTC TCG TTT GAT AAA TTG AAC AAT TAC TTA      1872
   Lys Thr Gly Thr Cys Pro Phe Ser Phe Asp Lys Leu Asn Asn Tyr Leu
       610                 615                 620
   ACT TTT AAC AAG TTC TGT TTG TCA TTG AAT CCT GTT GGT GCC AAC TGC      1920
   Thr Phe Asn Lys Phe Cys Leu Ser Leu Asn Pro Val Gly Ala Asn Cys
   625                 630                 635                 640
   AAG TTT GAT GTT GCC GCT CGT ACA AGA ACC AAT GAG CAG GTT GTT AGA      1968
   Lys Phe Asp Val Ala Ala Arg Thr Arg Thr Asn Glu Gln Val Val Arg
                   645                 650                 655
   AGT TTA TAT GTA ATA TAT GAA GAA GGA GAC AAC ATA GTG GGT GTG CCG      2016
   Ser Leu Tyr Val Ile Tyr Glu Glu Gly Asp Asn Ile Val Gly Val Pro
               660                 665                 670
   TCT GAC AAT AGT GGT CTT CAC GAC TTG TCA GTG CTA CAC TTA GAC TCC      2064
   Ser Asp Asn Ser Gly Leu His Asp Leu Ser Val Leu His Leu Asp Ser
           675                 680                 685
   TGT ACA GAT TAT AAT ATA TAT GGT AGA ACT GGT GTT GGT ATT ATT AGA      2112
   Cys Thr Asp Tyr Asn Ile Tyr Gly Arg Thr Gly Val Gly Ile Ile Arg
       690                 695                 700
   CAA ACT AAC AGT ACG CTA CTT AGT GGC TTA TAT TAC ACA TCA CTA TCA      2160
   Gln Thr Asn Ser Thr Leu Leu Ser Gly Leu Tyr Tyr Thr Ser Leu Ser
   705                 710                 715                 720
   GGT GAC TTG TTA GGG TTT AAA AAT GTT AGT GAT GGT GTC ATC TAT TCT      2208
   Gly Asp Leu Leu Gly Phe Lys Asn Val Ser Asp Gly Val Ile Tyr Ser
                   725                 730                 735
   GTC ACG CCA TGT GAT GTA AGC GCA CAA GCT GCT GTT ATT GAT GGC GCC        2256
   Val Thr Pro Cys Asp Val Ser Ala Gln Ala Ala Val Ile Asp Gly Ala
               740                 745                 750
   ATA GTT GGA GCT ATG ACT TCC ATT AAT AGT GAA ATG TTA GGT CTA ACA        2304
   Ile Val Gly Ala Met Thr Ser Ile Asn Ser Glu Met Leu Gly Leu Thr
           755                 760                 765
   CAT TGG ACA ACA ACA CCT AAT TTT TAT TAT TAT TCT ATA TAT AAT TAT        2352
   His Trp Thr Thr Thr Pro Asn Phe Tyr Tyr Tyr Ser Ile Tyr Asn Tyr
       770                 775                 780
   ACC AAT GAA AGG ACT CGT GGC ACA GCA ATT GAT AGT AAC GAT GTT GAT        2400
   Thr Asn Glu Arg Thr Arg Gly Thr Ala Ile Asp Ser Asn Asp Val Asp
   785                 790                 795                 800
   TGT GAA CCT ATC ATA ACC TAT TCT AAT ATA GGT GTT TGT AAA AAT GGA        2448
   Cys Glu Pro Ile Ile Thr Tyr Ser Asn Ile Gly Val Cys Lys Asn Gly
                   805                 810                 815
   GCT TTG GTT TTT ATT AAC GTC ACA CAT TCT GAT GGA GAC GTT CAA CCA        2496
   Ala Leu Val Phe Ile Asn Val Thr His Ser Asp Gly Asp Val Gln Pro
               820                 825                 830
   ATT AGC ACC GGT AAT GTC ACG ATA CCT ACA AAT TTT ACC ATA TCT GTG        2544
   Ile Ser Thr Gly Asn Val Thr Ile Pro Thr Asn Phe Thr Ile Ser Val
           835                 840                 845
   CAA GTT GAG TAC ATT CAG GTT TAC ACT ACA CCG GTG TCA ATA GAT TGT        2592
   Gln Val Glu Tyr Ile Gln Val Tyr Thr Thr Pro Val Ser Ile Asp Cys
       850                 855                 860
   TCA AGG TAC GTT TGC AAT GGT AAC CCT AGA TGC AAT AAA TTG TTA ACG        2640
   Ser Arg Tyr Val Cys Asn Gly Asn Pro Arg Cys Asn Lys Leu Leu Thr
   865                 870                 875                 880
   CAA TAC GTT TCT GCA TGT CAA ACT ATT GAG CAA GCA CTT GCA ATG GGT        2688
   Gln Tyr Val Ser Ala Cys Gln Thr Ile Glu Gln Ala Leu Ala Met Gly
                   885                 890                 895
   GCC AGA CTT GAA AAC ATG GAG ATT GAT TCC ATG TTG TTT GTT TCG GAA        2736
   Ala Arg Leu Glu Asn Met Glu Ile Asp Ser Met Leu Phe Val Ser Glu
               900                 905                 910
   AAT GCC CTT AAA TTG GCA TCT GTT GAA GCA TTC AAT AGT ACG GAA ACT        2784
   Asn Ala Leu Lys Leu Ala Ser Val Glu Ala Phe Asn Ser Thr Glu Thr
           915                 920                 925
   TTA GAT CCT ATT TAC AAA GAA TGG CCT AAC ATT GGT GGT TCT TGG CTA        2832
   Leu Asp Pro Ile Tyr Lys Glu Trp Pro Asn Ile Gly Gly Ser Trp Leu
       930                 935                 940
   GGA GGT TTA AAA GAC ATA TTG CCA TCT CAC AAC AGC AAA CGT AAG TAC        2880
   Gly Gly Leu Lys Asp Ile Leu Pro Ser His Asn Ser Lys Arg Lys Tyr
   945                 950                 955                 960
   CGG TCG GCT ATA GAA GAT TTG CTT TTT GAT AAG GTT GTA ACA TCT GGC        2928
   Arg Ser Ala Ile Glu Asp Leu Leu Phe Asp Lys Val Val Thr Ser Gly
                   965                 970                 975
   TTA GGT ACA GTT GAT GAA GAT TAT AAA CGT TGT ACA GGT GGT TAT GAC        2976
   Leu Gly Thr Val Asp Glu Asp Tyr Lys Arg Cys Thr Gly Gly Tyr Asp
               980                 985                 990
   ATA GCT GAC TTA GTG TGT GCA CAA TAT TAC AAT GGC ATC ATG GTG CTA      3024
   Ile Ala Asp Leu Val Cys Ala Gln Tyr Tyr Asn Gly Ile Met Val Leu
           995                 1000                1005
   CCT GGT GTA GCT AAT GAT GAC AAG ATG GCT ATG TAC ACT GCA TCT CTT      3072
   Pro Gly Val Ala Asn Asp Asp Lys Met Ala Met Tyr Thr Ala Ser Leu
       1010                1015                1020
   GCA GGT GGT ATA ACA TTA GGT GCA CTT GGT GGT GGC GCA GTG TCT ATA      3120
   Ala Gly Gly Ile Thr Leu Gly Ala Leu Gly Gly Gly Ala Val Ser Ile
   1025                1030                1035                1040
   CCT TTT GCA ATA GCA GTT CAA GCC AGA CTT AAT TAT GTT GCT CTA CAA      3168
   Pro Phe Ala Ile Ala Val Gln Ala Arg Leu Asn Tyr Val Ala Leu Gln
                   1045                1050                1055
   ACT GAT GTA TTG AGC AAG AAC CAG CAG ATC CTG GCT AAT GCT TTC AAT      3216
   Thr Asp Val Leu Ser Lys Asn Gln Gln Ile Leu Ala Asn Ala Phe Asn
               1060                1065                1070
   CAA GCT ATT GGT AAC ATT ACA CAG GCA TTT GGT AAG GTT AAT GAT GCT      3264
   Gln Ala Ile Gly Asn Ile Thr Gln Ala Phe Gly Lys Val Asn Asp Ala
           1075                1080                1085
   ATA CAT CAA ACG TCA CAA GGT CTT GCT ACT GTT GCT AAA GCA TTG GCA      3312
   Ile His Gln Thr Ser Gln Gly Leu Ala Thr Val Ala Lys Ala Leu Ala
       1090                1095                1100
   AAA GTG CAA GAT GTT GTT AAC ACA CAA GGG CAA GCT TTA AGC CAC CTA      3360
   Lys Val Gln Asp Val Val Asn Thr Gln Gly Gln Ala Leu Ser His Leu
   1105                1110                1115                1120
   ACA GTA CAA TTG CAA AAT AAT TTC CAA GCC ATT AGT AGT TCC ATT AGT      3408
   Thr Val Gln Leu Gln Asn Asn Phe Gln Ala Ile Ser Ser Ser Ile Ser
                   1125                1130                1135
   GAC ATT TAT AAC AGG CTT GAT GAA TTG AGT GCT GAT GCA CAA GTT GAC      3456
   Asp Ile Tyr Asn Arg Leu Asp Glu Leu Ser Ala Asp Ala Gln Val Asp
               1140                1145                1150
   AGG CTG ATT ACA GGA AGA CTT ACA GCA CTT AAT GCA TTT GTG TCT CAG      3504
   Arg Leu Ile Thr Gly Arg Leu Thr Ala Leu Asn Ala Phe Val Ser Gln
           1155                1160                1165
   ACT TTA ACC AGA CAA GCA GAG GTT AGG GCT AGC AGA CAG CTT GCT AAA      3552
   Thr Leu Thr Arg Gln Ala Glu Val Arg Ala Ser Arg Gln Leu Ala Lys
       1170                1175                1180
   GAC AAG GTA AAT GAA TGC GTT AGG TCT CAA TCT CAG AGA TTT GGA TTC      3600
   Asp Lys Val Asn Glu Cys Val Arg Ser Gln Ser Gln Arg Phe Gly Phe
   1185                1190                1195                1200
   TGT GGT AAT GGT ACA CAT TTA TTT TCA CTT GCA AAT GCA GCA CCA AAT      3648
   Cys Gly Asn Gly Thr His Leu Phe Ser Leu Ala Asn Ala Ala Pro Asn
                   1205                1210                1215
   GGC ATG ATC TTC TTT CAC ACA GTG CTA TTA CCA ACA GCT TAT GAA ACC      3696
   Gly Met Ile Phe Phe His Thr Val Leu Leu Pro Thr Ala Tyr Glu Thr
               1220                1225                1230
   GTG ACG GCC TGG TCA GGT ATT TGT GCA TCA GAT GGC GAT CGT ACT TTT      3744
   Val Thr Ala Trp Ser Gly Ile Cys Ala Ser Asp Gly Asp Arg Thr Phe
           1235                1240                1245
   GGA CTT GTT GTT AAG GAT GTC CAG TTG ACG CTG TTT CGC AAT CTA GAT        3792
   Gly Leu Val Val Lys Asp Val Gln Leu Thr Leu Phe Arg Asn Leu Asp
       1250                1255                1260
   GAC AAA TTC TAT TTG ACT CCC AGA ACT ATG TAT CAG CCT AGA GTT GCA        3840
   Asp Lys Phe Tyr Leu Thr Pro Arg Thr Met Tyr Gln Pro Arg Val Ala
   1265                1270                1275                1280
   ACT AGT TCT GAT TTT GTT CAA ATT GAA GGA TGT GAT GTG TTG TTT GTT        3888
   Thr Ser Ser Asp Phe Val Gln Ile Glu Gly Cys Asp Val Leu Phe Val
                   1285                1290                1295
   AAT GCA ACT GTA ATT GAC TTG CCT AGT ATT ATA CCT GAC TAT ATT GAT        3936
   Asn Ala Thr Val Ile Asp Leu Pro Ser Ile Ile Pro Asp Tyr Ile Asp
               1300                1305                1310
   ATT AAT CAA ACT GTT CAG GAC ATA TTA GAA AAT TTC AGA CCA AAT TGG        3984
   Ile Asn Gln Thr Val Gln Asp Ile Leu Glu Asn Phe Arg Pro Asn Trp
           1315                1320                1325
   ACT GTA CCT GAG TTG CCA CTT GAC ATT TTC AAT GCA ACC TAC TTA AAC        4032
   Thr Val Pro Glu Leu Pro Leu Asp Ile Phe Asn Ala Thr Tyr Leu Asn
       1330                1335                1340
   CTG ACT GGT GAA ATT AAT GAC TTA GAA TTT AGG TCA GAA AAG TTA CAT        4080
   Leu Thr Gly Glu Ile Asn Asp Leu Glu Phe Arg Ser Glu Lys Leu His
   1345                1350                1355                1360
   AAC ACC ACA GTA GAA CTT GCT ATT CTC ATT GAT AAT ATT AAT AAC ACA        4128
   Asn Thr Thr Val Glu Leu Ala Ile Leu Ile Asp Asn Ile Asn Asn Thr
                   1365                1370                1375
   TTA GTC AAT CTT GAA TGG CTC AAT AGA ATT GAA ACT TAT GTA AAA TGG        4176
   Leu Val Asn Leu Glu Trp Leu Asn Arg Ile Glu Thr Tyr Val Lys Trp
               1380                1385                1390
   CCT TGG TAT GTG TGG CTA CTA ATT GGA TTA GTA GTA ATA TTC TGC ATA        4224
   Pro Trp Tyr Val Trp Leu Leu Ile Gly Leu Val Val Ile Phe Cys Ile
           1395                1400                1405
   CCC ATA TTG CTA TTT TGT TGT TGT AGC ACT GGT TGT TGT GGA TGT ATT        4272
   Pro Ile Leu Leu Phe Cys Cys Cys Ser Thr Gly Cys Cys Gly Cys Ile
       1410                1415                1420
   GGG TGT TTA GGA AGC TGT TGT CAT TCC ATA TGT AGT AGA AGG CGA TTT        4320
   Gly Cys Leu Gly Ser Cys Cys His Ser Ile Cys Ser Arg Arg Arg Phe
   1425                1430                1435                1440
   GAA AGT TAT GAA CCA ATT GAA AAA GTG CAT GTC CAC TAA                    4359
   Glu Ser Tyr Glu Pro Ile Glu Lys Val His Val His
                   1445                1450
(2) INFORMATION FOR SEQ ID NO:2:
   (i) SEQUENCE CHARACTERISTICS:
      (A) LENGTH: 1452 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Ile Val Leu Val Thr Cys Leu Leu Phe Ser Tyr Asn Ser Val Ile
    1               5                  10                  15
   Cys Thr Ser Asn Asn Asp Cys Val Gln Val Asn Val Thr Gln Leu Pro
               20                  25                  30
   Gly Asn Glu Asn Ile Ile Lys Asp Phe Leu Phe His Thr Phe Lys Glu
           35                  40                  45
   Glu Gly Ser Val Val Val Gly Gly Tyr Tyr Pro Thr Glu Val Trp Tyr
       50                  55                  60
   Asn Cys Ser Arg Ser Ala Thr Thr Thr Ala Tyr Lys Asp Phe Ser Asn
   65                  70                  75                  80
   Ile His Ala Phe Tyr Phe Asp Met Glu Ala Met Glu Asn Ser Thr Gly
                   85                  90                  95
   Asn Ala Arg Gly Lys Pro Leu Leu Val His Val His Gly Asp Pro Val
               100                 105                 110
   Ser Ile Ile Ile Tyr Ile Ser Ala Tyr Arg Asp Asp Val Gln Gly Arg
           115                 120                 125
   Pro Leu Leu Lys His Gly Leu Leu Cys Ile Thr Lys Asn Lys Ile Ile
       130                 135                 140
   Asp Tyr Asn Thr Phe Thr Ser Ala Gln Trp Ser Ala Ile Cys Leu Gly
   145                 150                 155                 160
   Asp Asp Arg Lys Ile Pro Phe Ser Val Ile Pro Thr Gly Asn Gly Thr
                   165                 170                 175
   Lys Ile Phe Gly Leu Glu Trp Asn Asp Asp Tyr Val Thr Ala Tyr Ile
               180                 185                 190
   Ser Asp Arg Ser His His Leu Asn Ile Asn Asn Asn Trp Phe Asn Asn
           195                 200                 205
   Val Thr Ile Leu Tyr Ser Arg Ser Ser Thr Ala Thr Trp Gln Lys Ser
       210                 215                 220
   Ala Ala Tyr Val Tyr Gln Gly Val Ser Asn Phe Thr Tyr Tyr Lys Leu
   225                 230                 235                 240
   Asn Asn Thr Asn Gly Leu Lys Ser Tyr Glu Leu Cys Glu Asp Tyr Glu
                   245                 250                 255
   Cys Cys Thr Gly Tyr Ala Thr Asn Val Phe Ala Pro Thr Val Gly Gly
               260                 265                 270
   Tyr Ile Pro Asp Gly Phe Ser Phe Asn Asn Trp Phe Met Leu Thr Asn
           275                 280                 285
   Ser Ser Thr Phe Val Ser Gly Arg Phe Val Thr Asn Gln Pro Leu Leu
       290                 295                 300
   Val Asn Cys Leu Trp Pro Val Pro Ser Leu Gly Val Ala Ala Gln Glu
   305                 310                 315                 320
   Phe Cys Phe Glu Gly Ala Gln Phe Ser Gln Cys Asn Gly Val Ser Leu
                   325                 330                 335
   Asn Asn Thr Val Asp Val Ile Arg Phe Asn Leu Asn Phe Thr Thr Asp
               340                 345                 350
   Val Gln Ser Gly Met Gly Ala Thr Val Phe Ser Leu Asn Thr Thr Gly
           355                 360                 365
   Gly Val Ile Leu Glu Ile Ser Cys Tyr Asn Asp Thr Val Ser Glu Ser
       370                 375                 380
   Ser Phe Tyr Ser Tyr Gly Glu Ile Ser Phe Gly Val Thr Asp Gly Pro
   385                 390                 395                 400
   Arg Tyr Cys Tyr Ala Leu Tyr Asn Gly Thr Ala Leu Lys Tyr Leu Gly
                   405                 410                 415
   Thr Leu Pro Pro Ser Val Lys Glu Ile Ala Ile Ser Lys Trp Gly His
               420                 425                 430
   Phe Tyr Ile Asn Gly Tyr Asn Phe Phe Ser Thr Phe Pro Ile Asp Cys
           435                 440                 445
   Ile Ser Phe Asn Leu Thr Thr Gly Asp Ser Gly Ala Phe Trp Thr Ile
       450                 455                 460
   Ala Tyr Thr Ser Tyr Thr Asp Ala Leu Val Gln Val Glu Asn Thr Ala
   465                 470                 475                 480
   Ile Lys Lys Val Thr Tyr Cys Asn Ser His Ile Asn Asn Ile Lys Cys
                   485                 490                 495
   Ser Gln Leu Thr Ala Asn Leu Gln Asn Gly Phe Tyr Pro Val Ala Ser
               500                 505                 510
   Ser Glu Val Gly Leu Val Asn Lys Ser Val Val Leu Leu Pro Ser Phe
           515                 520                 525
   Tyr Ser His Thr Ser Val Asn Ile Thr Ile Asp Leu Gly Met Lys Arg
       530                 535                 540
   Ser Gly Tyr Gly Gln Pro Ile Ala Ser Thr Leu Ser Asn Ile Thr Leu
   545                 550                 555                 560
   Pro Met Gln Asp Asn Asn Thr Asp Val Tyr Cys Ile Arg Ser Asn Gln
                   565                 570                 575
   Phe Ser Val Tyr Val His Ser Thr Cys Lys Ser Ser Leu Trp Asp Asp
               580                 585                 590
   Val Phe Asn Ser Asp Cys Thr Asp Val Leu Tyr Ala Thr Ala Val Ile
           595                 600                 605
   Lys Thr Gly Thr Cys Pro Phe Ser Phe Asp Lys Leu Asn Asn Tyr Leu
       610                 615                 620
   Thr Phe Asn Lys Phe Cys Leu Ser Leu Asn Pro Val Gly Ala Asn Cys
   625                 630                 635                 640
   Lys Phe Asp Val Ala Ala Arg Thr Arg Thr Asn Glu Gln Val Val Arg
                   645                 650                 655
   Ser Leu Tyr Val Ile Tyr Glu Glu Gly Asp Asn Ile Val Gly Val Pro
               660                 665                 670
   Ser Asp Asn Ser Gly Leu His Asp Leu Ser Val Leu His Leu Asp Ser
           675                 680                 685
   Cys Thr Asp Tyr Asn Ile Tyr Gly Arg Thr Gly Val Gly Ile Ile Arg
       690                 695                 700
   Gln Thr Asn Ser Thr Leu Leu Ser Gly Leu Tyr Tyr Thr Ser Leu Ser
   705                 710                 715                 720
   Gly Asp Leu Leu Gly Phe Lys Asn Val Ser Asp Gly Val Ile Tyr Ser
                   725                 730                 735
   Val Thr Pro Cys Asp Val Ser Ala Gln Ala Ala Val Ile Asp Gly Ala
               740                 745                 750
   Ile Val Gly Ala Met Thr Ser Ile Asn Ser Glu Met Leu Gly Leu Thr
           755                 760                 765
   His Trp Thr Thr Thr Pro Asn Phe Tyr Tyr Tyr Ser Ile Tyr Asn Tyr
       770                 775                 780
   Thr Asn Glu Arg Thr Arg Gly Thr Ala Ile Asp Ser Asn Asp Val Asp
   785                 790                 795                 800
   Cys Glu Pro Ile Ile Thr Tyr Ser Asn Ile Gly Val Cys Lys Asn Gly
                   805                 810                 815
   Ala Leu Val Phe Ile Asn Val Thr His Ser Asp Gly Asp Val Gln Pro
               820                 825                 830
   Ile Ser Thr Gly Asn Val Thr Ile Pro Thr Asn Phe Thr Ile Ser Val
           835                 840                 845
   Gln Val Glu Tyr Ile Gln Val Tyr Thr Thr Pro Val Ser Ile Asp Cys
       850                 855                 860
   Ser Arg Tyr Val Cys Asn Gly Asn Pro Arg Cys Asn Lys Leu Leu Thr
   865                 870                 875                 880
   Gln Tyr Val Ser Ala Cys Gln Thr Ile Glu Gln Ala Leu Ala Met Gly
                   885                 890                 895
   Ala Arg Leu Glu Asn Met Glu Ile Asp Ser Met Leu Phe Val Ser Glu
               900                 905                 910
   Asn Ala Leu Lys Leu Ala Ser Val Glu Ala Phe Asn Ser Thr Glu Thr
           915                 920                 925
   Leu Asp Pro Ile Tyr Lys Glu Trp Pro Asn Ile Gly Gly Ser Trp Leu
       930                 935                 940
   Gly Gly Leu Lys Asp Ile Leu Pro Ser His Asn Ser Lys Arg Lys Tyr
   945                 950                 955                 960
   Arg Ser Ala Ile Glu Asp Leu Leu Phe Asp Lys Val Val Thr Ser Gly
                   965                 970                 975
   Leu Gly Thr Val Asp Glu Asp Tyr Lys Arg Cys Thr Gly Gly Tyr Asp
               980                 985                 990
   Ile Ala Asp Leu Val Cys Ala Gln Tyr Tyr Asn Gly Ile Met Val Leu
           995                 1000                1005
   Pro Gly Val Ala Asn Asp Asp Lys Met Ala Met Tyr Thr Ala Ser Leu
       1010                1015                1020
   Ala Gly Gly Ile Thr Leu Gly Ala Leu Gly Gly Gly Ala Val Ser Ile
   1025                1030                1035                1040
   Pro Phe Ala Ile Ala Val Gln Ala Arg Leu Asn Tyr Val Ala Leu Gln
                   1045                1050                1055
   Thr Asp Val Leu Ser Lys Asn Gln Gln Ile Leu Ala Asn Ala Phe Asn
               1060                1065                1070
   Gln Ala Ile Gly Asn Ile Thr Gln Ala Phe Gly Lys Val Asn Asp Ala
           1075                1080                1085
   Ile His Gln Thr Ser Gln Gly Leu Ala Thr Val Ala Lys Ala Leu Ala
       1090                1095                1100
   Lys Val Gln Asp Val Val Asn Thr Gln Gly Gln Ala Leu Ser His Leu
   1105                1110                1115                1120
   Thr Val Gln Leu Gln Asn Asn Phe Gln Ala Ile Ser Ser Ser Ile Ser
                   1125                1130                1135
   Asp Ile Tyr Asn Arg Leu Asp Glu Leu Ser Ala Asp Ala Gln Val Asp
               1140                1145                1150
   Arg Leu Ile Thr Gly Arg Leu Thr Ala Leu Asn Ala Phe Val Ser Gln
           1155                1160                1165
   Thr Leu Thr Arg Gln Ala Glu Val Arg Ala Ser Arg Gln Leu Ala Lys
       1170                1175                1180
   Asp Lys Val Asn Glu Cys Val Arg Ser Gln Ser Gln Arg Phe Gly Phe
   1185                1190                1195                1200
   Cys Gly Asn Gly Thr His Leu Phe Ser Leu Ala Asn Ala Ala Pro Asn
                   1205                1210                1215
   Gly Met Ile Phe Phe His Thr Val Leu Leu Pro Thr Ala Tyr Glu Thr
               1220                1225                1230
   Val Thr Ala Trp Ser Gly Ile Cys Ala Ser Asp Gly Asp Arg Thr Phe
           1235                1240                1245
   Gly Leu Val Val Lys Asp Val Gln Leu Thr Leu Phe Arg Asn Leu Asp
       1250                1255                1260
   Asp Lys Phe Tyr Leu Thr Pro Arg Thr Met Tyr Gln Pro Arg Val Ala
   1265                1270                1275                1280
   Thr Ser Ser Asp Phe Val Gln Ile Glu Gly Cys Asp Val Leu Phe Val
                   1285                1290                1295
   Asn Ala Thr Val Ile Asp Leu Pro Ser Ile Ile Pro Asp Tyr Ile Asp
               1300                1305                1310
   Ile Asn Gln Thr Val Gln Asp Ile Leu Glu Asn Phe Arg Pro Asn Trp
           1315                1320                1325
   Thr Val Pro Glu Leu Pro Leu Asp Ile Phe Asn Ala Thr Tyr Leu Asn
       1330                1335                1340
   Leu Thr Gly Glu Ile Asn Asp Leu Glu Phe Arg Ser Glu Lys Leu His
   1345                1350                1355                1360
   Asn Thr Thr Val Glu Leu Ala Ile Leu Ile Asp Asn Ile Asn Asn Thr
                   1365                1370                1375
   Leu Val Asn Leu Glu Trp Leu Asn Arg Ile Glu Thr Tyr Val Lys Trp
               1380                1385                1390
   Pro Trp Tyr Val Trp Leu Leu Ile Gly Leu Val Val Ile Phe Cys Ile
           1395                1400                1405
   Pro Ile Leu Leu Phe Cys Cys Cys Ser Thr Gly Cys Cys Gly Cys Ile
       1410                1415                1420
   Gly Cys Leu Gly Ser Cys Cys His Ser Ile Cys Ser Arg Arg Arg Phe
   1425                1430                1435                1440
   Glu Ser Tyr Glu Pro Ile Glu Lys Val His Val His
                   1445                1450
(2) INFORMATION FOR SEQ ID NO:3:
   (i) SEQUENCE CHARACTERISTICS:
      (A) LENGTH: 201 amino acids
      (B) TYPE: amino acid
      (D) TOPOLOGY: unknown
   (ii) MOLECULE TYPE: protein
   (xi) SEQUENCE DESCRIPTION: SEQ ID NO:3:
   Gly Ser Val Val Val Gly Gly Tyr Tyr Pro Thr Glu Val Trp Tyr Asn
   1               5                   10                  15
   Cys Ser Arg Ser Ala Thr Thr Thr Ala Tyr Lys Asp Phe Ser Asn Ile
               20                  25                  30
   His Ala Phe Tyr Phe Asp Met Glu Ala Met Glu Asn Ser Thr Gly Asn
           35                  40                  45
   Ala Arg Gly Lys Pro Leu Leu Val His Val His Gly Asp Pro Val Ser
       50                  55                  60
   Ile Ile Ile Tyr Ile Ser Ala Tyr Arg Asp Asp Val Gln Gly Arg Pro
   65                  70                  75                  80
   Leu Leu Lys His Gly Leu Leu Cys Ile Thr Lys Asn Lys Ile Ile Asp
                   85                  90                  95
   Tyr Asn Thr Phe Thr Ser Ala Gln Trp Ser Ala Ile Cys Leu Gly Asp
               100                 105                 110
   Asp Arg Lys Ile Pro Phe Ser Val Ile Pro Thr Gly Asn Gly Thr Lys
           115                 120                 125
   Ile Phe Gly Leu Glu Trp Asn Asp Asp Tyr Val Thr Ala Tyr Ile Ser
       130                 135                 140
   Asp Arg Ser His His Leu Asn Ile Asn Asn Asn Trp Phe Asn Asn Val
   145                 150                 155                 160
   Thr Ile Leu Tyr Ser Arg Ser Ser Thr Ala Thr Trp Gln Lys Ser Ala
                   165                 170                 175
   Ala Tyr Val Tyr Gln Gly Val Ser Asn Phe Thr Tyr Tyr Lys Leu Asn
               180                 185                 190
   Asn Thr Asn Gly Leu Lys Ser Tyr Glu
           195                 200
(2) INFORMATION FOR SEQ ID NO:4:
   (i) SEQUENCE CHARACTERISTICS:
      (A) LENGTH: 51 amino acids
      (B) TYPE: amino acid
      (D) TOPOLOGY: unknown
   (ii) MOLECULE TYPE: protein
   (xi) SEQUENCE DESCRIPTION: SEQ ID NO:4:
   Ser Cys Tyr Asn Asp Thr Val Ser Glu Ser Ser Phe Tyr Ser Tyr Gly
   1               5                   10                  15
   Glu Ile Ser Phe Gly Val Thr Asp Gly Pro Arg Tyr Cys Tyr Ala Leu
               20                  25                  30
   Tyr Asn Gly Thr Ala Leu Lys Tyr Leu Gly Thr Leu Pro Pro Ser Val
           35                  40                  45
   Lys Glu Ile
       50
(2) INFORMATION FOR SEQ ID NO:5:
   (i) SEQUENCE CHARACTERISTICS:
      (A) LENGTH: 21 amino acids
      (B) TYPE: amino acid
      (D) TOPOLOGY: unknown
   (ii) MOLECULE TYPE: protein
   (xi) SEQUENCE DESCRIPTION: SEQ ID NO:5:
   Ser Phe Asn Leu Thr Thr Gly Asp Ser Gly Ala Phe Trp Thr Ile Ala
   1               5                   10                  15
   Tyr Thr Ser Tyr Thr
               20
(2) INFORMATION FOR SEQ ID NO:6:
   (i) SEQUENCE CHARACTERISTICS:
      (A) LENGTH: 51 amino acids
      (B) TYPE: amino acid
      (D) TOPOLOGY: unknown
   (ii) MOLECULE TYPE: protein
   (xi) SEQUENCE DESCRIPTION: SEQ ID NO:6:
   Pro Ile Ala Ser Thr Leu Ser Asn Ile Thr Leu Pro Met Gln Asp Asn
   1               5                   10                  15
   Asn Thr Asp Val Tyr Cys Ile Arg Ser Asn Gln Phe Ser Val Tyr Val
               20                  25                  30
   His Ser Thr Cys Lys Ser Ser Leu Trp Asp Asp Val Phe Asn Ser Asp
           35                  40                  45
   Cys Thr Asp
       50
(2) INFORMATION FOR SEQ ID NO:7:
   (i) SEQUENCE CHARACTERISTICS:
      (A) LENGTH: 51 amino acids
      (B) TYPE: amino acid
      (D) TOPOLOGY: unknown
   (ii) MOLECULE TYPE: protein
   (xi) SEQUENCE DESCRIPTION: SEQ ID NO:7:
   Thr Asn Glu Gln Val Val Arg Ser Leu Tyr Val Ile Tyr Glu Glu Gly
   1               5                   10                  15
   Asp Asn Ile Val Gly Val Pro Ser Asp Asn Ser Gly Leu His Asp Leu
               20                  25                  30
   Ser Val Leu His Leu Asp Ser Cys Thr Asp Tyr Asn Ile Tyr Gly Arg
           35                  40                  45
   Thr Gly Val
       50
(2) INFORMATION FOR SEQ ID NO:8:
   (i) SEQUENCE CHARACTERISTICS:
      (A) LENGTH: 81 amino acids
      (B) TYPE: amino acid
      (D) TOPOLOGY: unknown
   (ii) MOLECULE TYPE: protein
   (xi) SEQUENCE DESCRIPTION: SEQ ID NO:8:
   Trp Thr Thr Thr Pro Asn Phe Tyr Tyr Tyr Ser Ile Tyr Asn Tyr Thr
   1               5                   10                  15
   Asn Glu Arg Thr Arg Gly Thr Ala Ile Asp Ser Asn Asp Val Asp Cys
               20                  25                  30
   Glu Pro Ile Ile Thr Tyr Ser Asn Ile Gly Val Cys Lys Asn Gly Ala
           35                  40                  45
   Leu Val Phe Ile Asn Val Thr His Ser Asp Gly Asp Val Gln Pro Ile
       50                  55                  60
   Ser Thr Gly Asn Val Thr Ile Pro Thr Asn Phe Thr Ile Ser Val Gln
   65                  70                  75                  80
   Val
(2) INFORMATION FOR SEQ ID NO:9:
   (i) SEQUENCE CHARACTERISTICS:
      (A) LENGTH: 126 amino acids
      (B) TYPE: amino acid
      (D) TOPOLOGY: unknown
   (ii) MOLECULE TYPE: protein
   (xi) SEQUENCE DESCRIPTION: SEQ ID NO:9:
   Glu Asn Met Glu Ile Asp Ser Met Leu Phe Val Ser Glu Asn Ala Leu
   1               5                   10                  15
   Lys Leu Ala Ser Val Glu Ala Phe Asn Ser Thr Glu Thr Leu Asp Pro
               20                  25                  30
   Ile Tyr Lys Glu Trp Pro Asn Ile Gly Gly Ser Trp Leu Gly Gly Leu
           35                  40                  45
   Lys Asp Ile Leu Pro Ser His Asn Ser Lys Arg Lys Tyr Arg Ser Ala
       50                  55                  60
   Ile Glu Asp Leu Leu Phe Asp Lys Val Val Thr Ser Gly Leu Gly Thr
   65                  70                  75                  80
   Val Asp Glu Asp Tyr Lys Arg Cys Thr Gly Gly Tyr Asp Ile Ala Asp
                   85                  90                  95
   Leu Val Cys Ala Gln Tyr Tyr Asn Gly Ile Met Val Leu Pro Gly Val
               100                 105                 110
   Ala Asn Asp Asp Lys Met Ala Met Tyr Thr Ala Ser Leu Ala
           115                 120                 125
(2) INFORMATION FOR SEQ ID NO:10:
   (i) SEQUENCE CHARACTERISTICS:
      (A) LENGTH: 76 amino acids
      (B) TYPE: amino acid
      (D) TOPOLOGY: unknown
   (ii) MOLECULE TYPE: protein
   (xi) SEQUENCE DESCRIPTION: SEQ ID NO:10:
   Gln Val Asp Arg Leu Ile Thr Gly Arg Leu Thr Ala Leu Asn Ala Phe
   1               5                   10                  15
   Val Ser Gln Thr Leu Thr Arg Gln Ala Glu Val Arg Ala Ser Arg Gln
               20                  25                  30
   Leu Ala Lys Asp Lys Val Asn Glu Cys Val Arg Ser Gln Ser Gln Arg
           35                  40                  45
   Phe Gly Phe Cys Gly Asn Gly Thr His Leu Phe Ser Leu Ala Asn Ala
       50                  55                  60
   Ala Pro Asn Gly Met Ile Phe Phe His Thr Val Leu
   65                  70                  75
(2) INFORMATION FOR SEQ ID NO:11:
   (i) SEQUENCE CHARACTERISTICS:
      (A) LENGTH: 203 amino acids
      (B) TYPE: amino acid
      (D) TOPOLOGY: unknown
   (ii) MOLECULE TYPE: protein
   (xi) SEQUENCE DESCRIPTION: SEQ ID NO:11:
   Leu Val Val Lys Asp Val Gln Leu Thr Leu Phe Arg Asn Leu Asp Asp
   1               5                   10                  15
   Lys Phe Tyr Leu Thr Pro Arg Thr Met Tyr Gln Pro Arg Val Ala Thr
               20                  25                  30
   Ser Ser Asp Phe Val Gln Ile Glu Gly Cys Asp Val Leu Phe Val Asn
           35                  40                  45
   Ala Thr Val Ile Asp Leu Pro Ser Ile Ile Pro Asp Tyr Ile Asp Ile
       50                  55                  60
   Asn Gln Thr Val Gln Asp Ile Leu Glu Asn Phe Arg Pro Asn Trp Thr
   65                  70                  75                  80
   Val Pro Glu Leu Pro Leu Asp Ile Phe Asn Ala Thr Tyr Leu Asn Leu
                   85                  90                  95
   Thr Gly Glu Ile Asn Asp Leu Glu Phe Arg Ser Glu Lys Leu His Asn
               100                 105                 110
   Thr Thr Val Glu Leu Ala Ile Leu Ile Asp Asn Ile Asn Asn Thr Leu
           115                 120                 125
   Val Asn Leu Glu Trp Leu Asn Arg Ile Glu Thr Tyr Val Lys Trp Pro
       130                 135                 140
   Trp Tyr Val Trp Leu Leu Ile Gly Leu Val Val Ile Phe Cys Ile Pro
   145                 150                 155                 160
   Ile Leu Leu Phe Cys Cys Cys Ser Thr Gly Cys Cys Gly Cys Ile Gly
                   165                 170                 175
   Cys Leu Gly Ser Cys Cys His Ser Ile Cys Ser Arg Arg Arg Phe Glu
               180                 185                 190
   Ser Tyr Glu Pro Ile Glu Lys Val His Val His
           195                 200
(2) INFORMATION FOR SEQ ID NO:12:
   (i) SEQUENCE CHARACTERISTICS:
      (A) LENGTH: 8 amino acids
      (B) TYPE: amino acid
      (D) TOPOLOGY: unknown
   (ii) MOLECULE TYPE: protein
   (xi) SEQUENCE DESCRIPTION: SEQ ID NO:12:
   Asp Phe Leu Phe His Thr Phe Lys
   1               5
(2) INFORMATION FOR SEQ ID NO:13:
   (i) SEQUENCE CHARACTERISTICS:
      (A) LENGTH: 19 amino acids
      (B) TYPE: amino acid
      (D) TOPOLOGY: unknown
   (ii) MOLECULE TYPE: protein
   (xi) SEQUENCE DESCRIPTION: SEQ ID NO:13:
   Trp Tyr Asn Cys Ser Arg Ser Ala Thr Thr Thr Ala Tyr Lys Asp Phe
   1               5                   10                  15
   Ser Asn Ile
(2) INFORMATION FOR SEQ ID NO:14:
   (i) SEQUENCE CHARACTERISTICS:
      (A) LENGTH: 5 amino acids
      (B) TYPE: amino acid
      (D) TOPOLOGY: unknown
   (ii) MOLECULE TYPE: protein
   (xi) SEQUENCE DESCRIPTION: SEQ ID NO:14:
   Tyr Val Thr Ala Tyr
   1               5
(2) INFORMATION FOR SEQ ID NO:15:
   (i) SEQUENCE CHARACTERISTICS:
      (A) LENGTH: 34 amino acids
      (B) TYPE: amino acid
      (D) TOPOLOGY: unknown
   (ii) MOLECULE TYPE: protein
   (xi) SEQUENCE DESCRIPTION: SEQ ID NO:15:
   Asn Asn Thr Asn Gly Leu Lys Ser Tyr Glu Leu Cys Glu Asp Tyr Glu
   1               5                   10                  15
   Cys Cys Thr Gly Tyr Ala Thr Asn Val Phe Ala Pro Thr Val Gly Gly
               20                  25                  30
   Tyr Ile
(2) INFORMATION FOR SEQ ID NO:16:
   (i) SEQUENCE CHARACTERISTICS:
      (A) LENGTH: 7 amino acids
      (B) TYPE: amino acid
      (D) TOPOLOGY: unknown
   (ii) MOLECULE TYPE: protein
   (xi) SEQUENCE DESCRIPTION: SEQ ID NO:16:
   Ser Leu Asn Asn Thr Val Asp
   1               5
(2) INFORMATION FOR SEQ ID NO:17:
   (i) SEQUENCE CHARACTERISTICS:
      (A) LENGTH: 34 amino acids
      (B) TYPE: amino acid
      (D) TOPOLOGY: unknown
   (ii) MOLECULE TYPE: protein
   (xi) SEQUENCE DESCRIPTION: SEQ ID NO:17:
   Gly Val Thr Asp Gly Pro Arg Tyr Cys Tyr Ala Leu Tyr Asn Gly Thr
   1               5                   10                  15
   Ala Leu Lys Tyr Leu Gly Thr Leu Pro Pro Ser Val Lys Glu Ile Ala
               20                  25                  30
   Ile Ser
(2) INFORMATION FOR SEQ ID NO:18:
   (i) SEQUENCE CHARACTERISTICS:
      (A) LENGTH: 27 amino acids
      (B) TYPE: amino acid
      (D) TOPOLOGY: unknown
   (ii) MOLECULE TYPE: protein
   (xi) SEQUENCE DESCRIPTION: SEQ ID NO:18:
   Ser Tyr Thr Asp Ala Leu Val Gln Val Glu Asn Thr Ala Ile Lys Lys
   1               5                   10                  15
   Val Thr Tyr Cys Asn Ser His Ile Asn Asn Ile
               20                  25
(2) INFORMATION FOR SEQ ID NO:19:
   (i) SEQUENCE CHARACTERISTICS:
      (A) LENGTH: 15 amino acids
      (B) TYPE: amino acid
      (D) TOPOLOGY: unknown
   (ii) MOLECULE TYPE: protein
   (xi) SEQUENCE DESCRIPTION: SEQ ID NO:19:
   Ile Ser Val Gln Val Glu Tyr Ile Gln Val Tyr Thr Thr Pro Val
   1               5                   10                  15
(2) INFORMATION FOR SEQ ID NO:20:
   (i) SEQUENCE CHARACTERISTICS:
      (A) LENGTH: 37 amino acids
      (B) TYPE: amino acid
      (D) TOPOLOGY: unknown
   (ii) MOLECULE TYPE: protein
   (xi) SEQUENCE DESCRIPTION: SEQ ID NO:20:
   Lys Leu Ala Ser Val Glu Ala Phe Asn Ser Thr Glu Thr Leu Asp Pro
   1               5                   10                  15
   Ile Tyr Lys Glu Trp Pro Asn Ile Gly Gly Ser Trp Leu Gly Gly Leu
               20                  25                  30
   Lys Asp Ile Leu Pro
           35
(2) INFORMATION FOR SEQ ID NO:21:
   (i) SEQUENCE CHARACTERISTICS:
      (A) LENGTH: 16 amino acids
      (B) TYPE: amino acid
      (D) TOPOLOGY: unknown
   (ii) MOLECULE TYPE: protein
   (xi) SEQUENCE DESCRIPTION: SEQ ID NO:21:
   Leu Gly Thr Val Asp Glu Asp Tyr Lys Arg Cys Thr Gly Gly Tyr Asp
   1               5                   10                  15
(2) INFORMATION FOR SEQ ID NO:22:
   (i) SEQUENCE CHARACTERISTICS:
      (A) LENGTH: 78 amino acids
      (B) TYPE: amino acid
      (D) TOPOLOGY: unknown
   (ii) MOLECULE TYPE: protein
   (xi) SEQUENCE DESCRIPTION: SEQ ID NO:22:
   Ala Asn Ala Phe Asn Gln Ala Ile Gly Asn Ile Thr Gln Ala Phe Gly
   1               5                   10                  15
   Lys Val Asn Asp Ala Ile His Gln Thr Ser Gln Gly Leu Ala Thr Val
               20                  25                  30
   Ala Lys Ala Leu Ala Lys Val Gln Asp Val Val Asn Thr Gln Gly Gln
           35                  40                  45
   Ala Leu Ser His Leu Thr Val Gln Leu Gln Asn Asn Phe Gln Ala Ile
       50                  55                  60
   Ser Ser Ser Ile Ser Asp Ile Tyr Asn Arg Leu Asp Glu Leu
   65                  70                  75
(2) INFORMATION FOR SEQ ID NO:23:
   (i) SEQUENCE CHARACTERISTICS:
      (A) LENGTH: 26 amino acids
      (B) TYPE: amino acid
      (D) TOPOLOGY: unknown
   (ii) MOLECULE TYPE: protein
   (xi) SEQUENCE DESCRIPTION: SEQ ID NO:23:
   Leu Ala Ile Leu Ile Asp Asn Ile Asn Asn Thr Leu Val Asn Leu Glu
   1               5                   10                  15
   Trp Leu Asn Arg Ile Glu Thr Tyr Val Lys
               20                  25
(2) INFORMATION FOR SEQ ID NO:24:
   (i) SEQUENCE CHARACTERISTICS:
      (A) LENGTH: 372 base pairs
      (B) TYPE: nucleic acid
      (C) STRANDEDNESS: double
      (D) TOPOLOGY: unknown
   (ii) MOLECULE TYPE: DNA (genomic)
   (ix) FEATURE:
      (A) NAME/KEY: CDS
      (B) LOCATION: 1..372
   (xi) SEQUENCE DESCRIPTION: SEQ ID NO:24:
   CAA GGG CAA GCT TTA AGC CAC CTA ACA GTA CAA TTG CAA AAT AAT TTC          48
   Gln Gly Gln Ala Leu Ser His Leu Thr Val Gln Leu Gln Asn Asn Phe
     1               5                  10                  15
   CAA GCC ATT AGT AGT TCC ATT AGT GAC ATT TAT AAC AGG CTT GAT GAA          96
   Gln Ala Ile Ser Ser Ser Ile Ser Asp Ile Tyr Asn Arg Leu Asp Glu
                20                  25                  30
   TTG AGT GCT GAT GCA CAA GTT GAC AGG CTG ATT ACA GGA AGA CTT ACA         144
   Leu Ser Ala Asp Ala Gln Val Asp Arg Leu Ile Thr Gly Arg Leu Thr
            35                  40                  45
   GCA CTT AAT GCA TTT GTG TCT CAG ACT TTA ACC AGA CAA GCA GAG GTT         192
   Ala Leu Asn Ala Phe Val Ser Gln Thr Leu Thr Arg Gln Ala Glu Val
        50                  55                  60
   AGG GCT AGC AGA CAG CTT GCT AAA GAC AAG GTA AAT GAA TGC GTT AGG         240
   Arg Ala Ser Arg Gln Leu Ala Lys Asp Lys Val Asn Glu Cys Val Arg
    65                  70                  75                  80
   TCT CAA TCT CAG AGA TTT GGA TTC TGT GGT AAT GGT ACA CAT TTA TTT         288
   Ser Gln Ser Gln Arg Phe Gly Phe Cys Gly Asn Gly Thr His Leu Phe
                    85                  90                  95
   TCA CTT GCA AAT GCA GCA CCA AAT GGC ATG ATC TTC TTT CAC ACA GTG         336
   Ser Leu Ala Asn Ala Ala Pro Asn Gly Met Ile Phe Phe His Thr Val
               100                 105                 110
   CTA TTA CCA ACA GCT TAT GAA ACC GTG ACG GCC TGG                         372
   Leu Leu Pro Thr Ala Tyr Glu Thr Val Thr Ala Trp
           115                 120
(2) INFORMATION FOR SEQ ID NO:25:
   (i) SEQUENCE CHARACTERISTICS:
      (A) LENGTH: 124 amino acids
      (B) TYPE: amino acid
      (D) TOPOLOGY: linear
   (ii) MOLECULE TYPE: protein
   (xi) SEQUENCE DESCRIPTION: SEQ ID NO:25:
   Gln Gly Gln Ala Leu Ser His Leu Thr Val Gln Leu Gln Asn Asn Phe
    1               5                  10                  15
   Gln Ala Ile Ser Ser Ser Ile Ser Asp Ile Tyr Asn Arg Leu Asp Glu
               20                  25                  30
   Leu Ser Ala Asp Ala Gln Val Asp Arg Leu Ile Thr Gly Arg Leu Thr
           35                  40                  45
   Ala Leu Asn Ala Phe Val Ser Gln Thr Leu Thr Arg Gln Ala Glu Val
       50                  55                  60
   Arg Ala Ser Arg Gln Leu Ala Lys Asp Lys Val Asn Glu Cys Val Arg
   65                  70                  75                  80
   Ser Gln Ser Gln Arg Phe Gly Phe Cys Gly Asn Gly Thr His Leu Phe
                   85                  90                  95
   Ser Leu Ala Asn Ala Ala Pro Asn Gly Met Ile Phe Phe His Thr Val
               100                 105                 110
   Leu Leu Pro Thr Ala Tyr Glu Thr Val Thr Ala Trp
           115                 120
(2) INFORMATION FOR SEQ ID NO:26:
   (i) SEQUENCE CHARACTERISTICS:
      (A) LENGTH: 180 base pairs
      (B) TYPE: nucleic acid
      (C) STRANDEDNESS: double
      (D) TOPOLOGY: unknown
   (ii) MOLECULE TYPE: DNA (genomic)
   (ix) FEATURE:
      (A) NAME/KEY: CDS
      (B) LOCATION: 1..180
   (xi) SEQUENCE DESCRIPTION: SEQ ID NO:26:
   CTT GGT ATG AAG CGT AGT GGT TAT GGT CAA CCC ATA GCC TCA ACA TTA         48
   Leu Gly Met Lys Arg Ser Gly Tyr Gly Gln Pro Ile Ala Ser Thr Leu
     1               5                  10                  15
   AGT AAC ATC ACA CTA CCA ATG CAG GAT AAT AAC ACC GAT GTG TAC TGC         96
   Ser Asn Ile Thr Leu Pro Met Gln Asp Asn Asn Thr Asp Val Tyr Cys
                20                  25                  30
   ATT CGT TCT AAC CAA TTT TCA GTT TAC GTT CAT TCC ACT TGT AAA AGT        144
   Ile Arg Ser Asn Gln Phe Ser Val Tyr Val His Ser Thr Cys Lys Ser
            35                  40                  45
   TCT TTA TGG GAC GAT GTG TTT AAT TCC GAC TGC ACA                        180
   Ser Leu Trp Asp Asp Val Phe Asn Ser Asp Cys Thr
        50                  55                  60
(2) INFORMATION FOR SEQ ID NO:27:
   (i) SEQUENCE CHARACTERISTICS:
      (A) LENGTH: 60 amino acids
      (B) TYPE: amino acid
      (D) TOPOLOGY: linear
   (ii) MOLECULE TYPE: protein
   (xi) SEQUENCE DESCRIPTION: SEQ ID NO:27:
   Leu Gly Met Lys Arg Ser Gly Tyr Gly Gln Pro Ile Ala Ser Thr Leu
     1               5                  10                  15
   Ser Asn Ile Thr Leu Pro Met Gln Asp Asn Asn Thr Asp Val Tyr Cys
                20                  25                  30
   Ile Arg Ser Asn Gln Phe Ser Val Tyr Val His Ser Thr Cys Lys Ser
            35                  40                  45
   Ser Leu Trp Asp Asp Val Phe Asn Ser Asp Cys Thr
        50                  55                  60
(2) INFORMATION FOR SEQ ID NO:28:
   (i) SEQUENCE CHARACTERISTICS:
      (A) LENGTH: 141 base pairs
      (B) TYPE: nucleic acid
      (C) STRANDEDNESS: double
      (D) TOPOLOGY: unknown
   (ii) MOLECULE TYPE: DNA (genomic)
   (ix) FEATURE:
      (A) NAME/KEY: CDS
      (B) LOCATION: 1..141
   (xi) SEQUENCE DESCRIPTION: SEQ ID NO:28:
   GTC ATT AGA TTC AAC CTT AAT TTT ACC ACA GAT GTA CAA TCT GGT ATG           48
   Val Ile Arg Phe Asn Leu Asn Phe Thr Thr Asp Val Gln Ser Gly Met
     1               5                  10                  15
   GGT GCT ACA GTA TTT TCA CTG AAT ACA ACA GGT GGT GTC ATT CTT GAG           96
   Gly Ala Thr Val Phe Ser Leu Asn Thr Thr Gly Gly Val Ile Leu Glu
                20                  25                  30
   ATT TCT TGT TAT AAT GAT ACA GTG AGT GAG TCA AGT TTC TAC AGT              141
   Ile Ser Cys Tyr Asn Asp Thr Val Ser Glu Ser Ser Phe Tyr Ser
            35                  40                  45
(2) INFORMATION FOR SEQ ID NO:29:
   (i) SEQUENCE CHARACTERISTICS:
      (A) LENGTH: 47 amino acids
      (B) TYPE: amino acid
      (D) TOPOLOGY: linear
   (ii) MOLECULE TYPE: protein
   (xi) SEQUENCE DESCRIPTION: SEQ ID NO:29:
   Val Ile Arg Phe Asn Leu Asn Phe Thr Thr Asp Val Gln Ser Gly Met
     1               5                  10                  15
   Gly Ala Thr Val Phe Ser Leu Asn Thr Thr Gly Gly Val Ile Leu Glu
                20                  25                  30
   Ile Ser Cys Tyr Asn Asp Thr Val Ser Glu Ser Ser Phe Tyr Ser
            35                  40                  45
(2) INFORMATION FOR SEQ ID NO:30:
   (i) SEQUENCE CHARACTERISTICS:
      (A) LENGTH: 51 base pairs
      (B) TYPE: nucleic acid
      (C) STRANDEDNESS: double
      (D) TOPOLOGY: unknown
   (ii) MOLECULE TYPE: DNA (genomic)
   (ix) FEATURE:
      (A) NAME/KEY: CDS
      (B) LOCATION: 1..51
   (xi) SEQUENCE DESCRIPTION: SEQ ID NO:30:
   TGT ATA ACT AAA AAT AAA ATC ATT GAC TAT AAC ACG TTT ACC AGC GCA         48
   Cys Ile Thr Lys Asn Lys Ile Ile Asp Tyr Asn Thr Phe Thr Ser Ala
     1               5                  10                  15
   CAG                                                                     51
   Gln
(2) INFORMATION FOR SEQ ID NO:31:
   (i) SEQUENCE CHARACTERISTICS:
      (A) LENGTH: 17 amino acids
      (B) TYPE: amino acid
      (D) TOPOLOGY: linear
   (ii) MOLECULE TYPE: protein
   (xi) SEQUENCE DESCRIPTION: SEQ ID NO:31:
   Cys Ile Thr Lys Asn Lys Ile Ile Asp Tyr Asn Thr Phe Thr Ser Ala
     1               5                  10                  15
   Gln
(2) INFORMATION FOR SEQ ID NO:32:
   (i) SEQUENCE CHARACTERISTICS:
      (A) LENGTH: 42 base pairs
      (B) TYPE: nucleic acid
      (C) STRANDEDNESS: double
      (D) TOPOLOGY: unknown
   (ii) MOLECULE TYPE: DNA (genomic)
   (ix) FEATURE:
      (A) NAME/KEY: CDS
      (B) LOCATION: 1..42
   (xi) SEQUENCE DESCRIPTION: SEQ ID NO:32:
   TCT TGT TAT AAT GAT ACA GTG AGT GAG TCA AGT TTC TAC AGT                   42
   Ser Cys Tyr Asn Asp Thr Val Ser Glu Ser Ser Phe Tyr Ser
     1               5                  10
(2) INFORMATION FOR SEQ ID NO:33:
   (i) SEQUENCE CHARACTERISTICS:
      (A) LENGTH: 14 amino acids
      (B) TYPE: amino acid
      (D) TOPOLOGY: linear
   (ii) MOLECULE TYPE: protein
   (xi) SEQUENCE DESCRIPTION: SEQ ID NO:33:
   Ser Cys Tyr Asn Asp Thr Val Ser Glu Ser Ser Phe Tyr Ser
     1               5                  10
(2) INFORMATION FOR SEQ ID NO:34:
   (i) SEQUENCE CHARACTERISTICS:
      (A) LENGTH: 51 base pairs
      (B) TYPE: nucleic acid
      (C) STRANDEDNESS: double
      (D) TOPOLOGY: unknown
   (ii) MOLECULE TYPE: DNA (genomic)
   (ix) FEATURE:
      (A) NAME/KEY: CDS
      (B) LOCATION: 1..51
   (xi) SEQUENCE DESCRIPTION: SEQ ID NO:34:
   ATT GGG TGT TTA GGA AGC TGT TGT CAT TCC ATA TGT AGT AGA AGG CGA        48
   Ile Gly Cys Leu Gly Ser Cys Cys His Ser Ile Cys Ser Arg Arg Arg
     1               5                  10                  15
   TTT                                                                    51
   Phe
(2) INFORMATION FOR SEQ ID NO:35:
   (i) SEQUENCE CHARACTERISTICS:
      (A) LENGTH: 17 amino acids
      (B) TYPE: amino acid
      (D) TOPOLOGY: linear
   (ii) MOLECULE TYPE: protein
   (xi) SEQUENCE DESCRIPTION: SEQ ID NO:35:
   Ile Gly Cys Leu Gly Ser Cys Cys His Ser Ile Cys Ser Arg Arg Arg
     1               5                  10                  15
   Phe
(2) INFORMATION FOR SEQ ID NO:36:
   (i) SEQUENCE CHARACTERISTICS:
      (A) LENGTH: 42 base pairs
      (B) TYPE: nucleic acid
      (C) STRANDEDNESS: double
      (D) TOPOLOGY: unknown
   (ii) MOLECULE TYPE: DNA (genomic)
   (ix) FEATURE:
      (A) NAME/KEY: CDS
      (B) LOCATION: 1..42
   (xi) SEQUENCE DESCRIPTION: SEQ ID NO:36:
   TGC ATA CCC ATA TTG CTA TTT TGT TGT TGT AGC ACT GGT TGT                42
   Cys Ile Pro Ile Leu Leu Phe Cys Cys Cys Ser Thr Gly Cys
     1               5                  10
(2) INFORMATION FOR SEQ ID NO:37:
   (i) SEQUENCE CHARACTERISTICS:
      (A) LENGTH: 14 amino acids
      (B) TYPE: amino acid
      (D) TOPOLOGY: linear
   (ii) MOLECULE TYPE: protein
   (xi) SEQUENCE DESCRIPTION: SEQ ID NO:37:
   Cys Ile Pro Ile Leu Leu Phe Cys Cys Cys Ser Thr Gly Cys
    1               5                  10
(2) INFORMATION FOR SEQ ID NO:38:
   (i) SEQUENCE CHARACTERISTICS:
      (A) LENGTH: 195 base pairs
      (B) TYPE: nucleic acid
      (C) STRANDEDNESS: double
      (D) TOPOLOGY: unknown
   (ii) MOLECULE TYPE: DNA (genomic)
   (ix) FEATURE:
      (A) NAME/KEY: CDS
      (B) LOCATION: 1..195
   (xi) SEQUENCE DESCRIPTION: SEQ ID NO:38:
   TAC TTA AAC CTG ACT GGT GAA ATT AAT GAC TTA GAA TTT AGG TCA GAA          48
   Tyr Leu Asn Leu Thr Gly Glu Ile Asn Asp Leu Glu Phe Arg Ser Glu
     1               5                  10                  15
   AAG TTA CAT AAC ACC ACA GTA GAA CTT GCT ATT CTC ATT GAT AAT ATT          96
   Lys Leu His Asn Thr Thr Val Glu Leu Ala Ile Leu Ile Asp Asn Ile
                20                  25                  30
   AAT AAC ACA TTA GTC AAT CTT GAA TGG CTC AAT AGA ATT GAA ACT TAT         144
   Asn Asn Thr Leu Val Asn Leu Glu Trp Leu Asn Arg Ile Glu Thr Tyr
            35                  40                  45
   GTA AAA TGG CCT TGG TAT GTG TGG CTA CTA ATT GGA TTA GTA GTA ATA         192
   Val Lys Trp Pro Trp Tyr Val Trp Leu Leu Ile Gly Leu Val Val Ile
        50                  55                  60
   TTC                                                                     195
   Phe
    65
(2) INFORMATION FOR SEQ ID NO:39:
   (i) SEQUENCE CHARACTERISTICS:
      (A) LENGTH: 65 amino acids
      (B) TYPE: amino acid
      (D) TOPOLOGY: linear
   (ii) MOLECULE TYPE: protein
   (xi) SEQUENCE DESCRIPTION: SEQ ID NO:39:
   Tyr Leu Asn Leu Thr Gly Glu Ile Asn Asp Leu Glu Phe Arg Ser Glu
    1               5                  10                  15
   Lys Leu His Asn Thr Thr Val Glu Leu Ala Ile Leu Ile Asp Asn Ile
               20                  25                  30
   Asn Asn Thr Leu Val Asn Leu Glu Trp Leu Asn Arg Ile Glu Thr Tyr
           35                  40                  45
   Val Lys Trp Pro Trp Tyr Val Trp Leu Leu Ile Gly Leu Val Val Ile
       50                  55                  60
   Phe
    65
(2) INFORMATION FOR SEQ ID NO:40:
   (i) SEQUENCE CHARACTERISTICS:
      (A) LENGTH: 765 base pairs
      (B) TYPE: nucleic acid
      (C) STRANDEDNESS: double
      (D) TOPOLOGY: unknown
   (ii) MOLECULE TYPE: DNA (genomic)
   (ix) FEATURE:
      (A) NAME/KEY: CDS
      (B) LOCATION: 1..765
   (xi) SEQUENCE DESCRIPTION: SEQ ID NO:40:
   GAT GGA CCG CGT TAC TGT TAC GCA CTC TAT AAT GGC ACG GCT CTT AAG          48
   Asp Gly Pro Arg Tyr Cys Tyr Ala Leu Tyr Asn Gly Thr Ala Leu Lys
     1               5                  10                  15
   TAT TTA GGA ACA TTA CCA CCT AGT GTC AAG GAA ATT GCT ATT AGT AAG          96
   Tyr Leu Gly Thr Leu Pro Pro Ser Val Lys Glu Ile Ala Ile Ser Lys
                20                  25                  30
   TGG GGC CAT TTT TAT ATT AAT GGT TAC AAT TTC TTT AGC ACT TTT CCT         144
   Trp Gly His Phe Tyr Ile Asn Gly Tyr Asn Phe Phe Ser Thr Phe Pro
            35                  40                  45
   ATT GAT TGT ATA TCT TTT AAT TTA ACC ACT GGT GAT AGT GGA GCA TTT         192
   Ile Asp Cys Ile Ser Phe Asn Leu Thr Thr Gly Asp Ser Gly Ala Phe
        50                  55                  60
   TGG ACA ATT GCT TAC ACA TCG TAC ACT GAC GCA TTA GTA CAA GTT GAA         240
   Trp Thr Ile Ala Tyr Thr Ser Tyr Thr Asp Ala Leu Val Gln Val Glu
    65                  70                  75                  80
   AAC ACA GCT ATT AAA AAG GTG ACG TAT TGT AAC AGT CAC ATT AAT AAC         288
   Asn Thr Ala Ile Lys Lys Val Thr Tyr Cys Asn Ser His Ile Asn Asn
                    85                  90                  95
   ATT AAA TGT TCT CAA CTT ACT GCT AAT TTG CAA AAT GGA TTT TAT CCT         336
   Ile Lys Cys Ser Gln Leu Thr Ala Asn Leu Gln Asn Gly Phe Tyr Pro
               100                 105                 110
   GTT GCT TCA AGT GAA GTT GGT CTT GTC AAT AAG AGT GTT GTG TTA CTA         384
   Val Ala Ser Ser Glu Val Gly Leu Val Asn Lys Ser Val Val Leu Leu
           115                 120                 125
   CCT AGT TTC TAT TCA CAT ACC AGT GTT AAT ATA ACT ATT GAT CTT GGT         432
   Pro Ser Phe Tyr Ser His Thr Ser Val Asn Ile Thr Ile Asp Leu Gly
       130                 135                 140
   ATG AAG CGT AGT GGT TAT GGT CAA CCC ATA GCC TCA ACA TTA AGT AAC         480
   Met Lys Arg Ser Gly Tyr Gly Gln Pro Ile Ala Ser Thr Leu Ser Asn
   145                 150                 155                 160
   ATC ACA CTA CCA ATG CAG GAT AAT AAC ACC GAT GTG TAC TGC ATT CGT         528
   Ile Thr Leu Pro Met Gln Asp Asn Asn Thr Asp Val Tyr Cys Ile Arg
                   165                 170                 175
   TCT AAC CAA TTT TCA GTT TAC GTT CAT TCC ACT TGT AAA AGT TCT TTA         576
   Ser Asn Gln Phe Ser Val Tyr Val His Ser Thr Cys Lys Ser Ser Leu
               180                 185                 190
   TGG GAC GAT GTG TTT AAT TCC GAC TGC ACA GAT GTT TTA TAT GCT ACA         624
   Trp Asp Asp Val Phe Asn Ser Asp Cys Thr Asp Val Leu Tyr Ala Thr
           195                 200                 205
   GCT GTT ATA AAA ACT GGT ACT TGT CCT TTC TCG TTT GAT AAA TTG AAC         672
   Ala Val Ile Lys Thr Gly Thr Cys Pro Phe Ser Phe Asp Lys Leu Asn
       210                 215                 220
   AAT TAC TTA ACT TTT AAC AAG TTC TGT TTG TCA TTG AAT CCT GTT GGT         720
   Asn Tyr Leu Thr Phe Asn Lys Phe Cys Leu Ser Leu Asn Pro Val Gly
   225                 230                 235                 240
   GCC AAC TGC AAG TTT GAT GTT GCC GCT CGT ACA AGA ACC AAT GAG             765
   Ala Asn Cys Lys Phe Asp Val Ala Ala Arg Thr Arg Thr Asn Glu
                   245                 250                 255
(2) INFORMATION FOR SEQ ID NO:41:
   (i) SEQUENCE CHARACTERISTICS:
      (A) LENGTH: 255 amino acids
      (B) TYPE: amino acid
      (D) TOPOLOGY: linear
   (ii) MOLECULE TYPE: protein
   (xi) SEQUENCE DESCRIPTION: SEQ ID NO:41:
   Asp Gly Pro Arg Tyr Cys Tyr Ala Leu Tyr Asn Gly Thr Ala Leu Lys
    1               5                  10                  15
   Tyr Leu Gly Thr Leu Pro Pro Ser Val Lys Glu Ile Ala Ile Ser Lys
               20                  25                  30
   Trp Gly His Phe Tyr Ile Asn Gly Tyr Asn Phe Phe Ser Thr Phe Pro
           35                  40                  45
   Ile Asp Cys Ile Ser Phe Asn Leu Thr Thr Gly Asp Ser Gly Ala Phe
       50                  55                  60
   Trp Thr Ile Ala Tyr Thr Ser Tyr Thr Asp Ala Leu Val Gln Val Glu
   65                  70                  75                  80
   Asn Thr Ala Ile Lys Lys Val Thr Tyr Cys Asn Ser His Ile Asn Asn
                   85                  90                  95
   Ile Lys Cys Ser Gln Leu Thr Ala Asn Leu Gln Asn Gly Phe Tyr Pro
               100                 105                 110
   Val Ala Ser Ser Glu Val Gly Leu Val Asn Lys Ser Val Val Leu Leu
           115                 120                 125
   Pro Ser Phe Tyr Ser His Thr Ser Val Asn Ile Thr Ile Asp Leu Gly
       130                 135                 140
   Met Lys Arg Ser Gly Tyr Gly Gln Pro Ile Ala Ser Thr Leu Ser Asn
   145                 150                 155                 160
   Ile Thr Leu Pro Met Gln Asp Asn Asn Thr Asp Val Tyr Cys Ile Arg
                   165                 170                 175
   Ser Asn Gln Phe Ser Val Tyr Val His Ser Thr Cys Lys Ser Ser Leu
               180                 185                 190
   Trp Asp Asp Val Phe Asn Ser Asp Cys Thr Asp Val Leu Tyr Ala Thr
           195                 200                 205
   Ala Val Ile Lys Thr Gly Thr Cys Pro Phe Ser Phe Asp Lys Leu Asn
       210                 215                 220
   Asn Tyr Leu Thr Phe Asn Lys Phe Cys Leu Ser Leu Asn Pro Val Gly
   225                 230                 235                 240
   Ala Asn Cys Lys Phe Asp Val Ala Ala Arg Thr Arg Thr Asn Glu
                   245                 250                 255
(2) INFORMATION FOR SEQ ID NO:42:
   (i) SEQUENCE CHARACTERISTICS:
      (A) LENGTH: 1284 base pairs
      (B) TYPE: nucleic acid
      (C) STRANDEDNESS: double
      (D) TOPOLOGY: unknown
   (ii) MOLECULE TYPE: DNA (genomic)
   (ix) FEATURE:
      (A) NAME/KEY: CDS
      (B) LOCATION: 1..1284
   (xi) SEQUENCE DESCRIPTION: SEQ ID NO:42:
   AGG CCT CTT TTA AAA CAT GGT TTG TTG TGT ATA ACT AAA AAT AAA ATC      48
   Arg Pro Leu Leu Lys His Gly Leu Leu Cys Ile Thr Lys Asn Lys Ile
    1               5                  10                  15
   ATT GAC TAT AAC ACG TTT ACC AGC GCA CAG TGG AGT GCC ATA TGT TTG      96
   Ile Asp Tyr Asn Thr Phe Thr Ser Ala Gln Trp Ser Ala Ile Cys Leu
               20                  25                  30
   GGT GAT GAC AGA AAA ATA CCA TTC TCT GTC ATA CCC ACA GGT AAT GGT     144
   Gly Asp Asp Arg Lys Ile Pro Phe Ser Val Ile Pro Thr Gly Asn Gly
           35                  40                  45
   ACA AAA ATA TTT GGT CTT GAG TGG AAT GAT GAC TAT GTT ACA GCC TAT     192
   Thr Lys Ile Phe Gly Leu Glu Trp Asn Asp Asp Tyr Val Thr Ala Tyr
       50                  55                  60
   ATT AGT GAT CGT TCT CAC CAT TTG AAC ATC AAT AAT AAT TGG TTT AAC     240
   Ile Ser Asp Arg Ser His His Leu Asn Ile Asn Asn Asn Trp Phe Asn
   65                  70                  75                  80
   AAT GTG ACA ATC CTA TAC TCT CGA TCA AGC ACT GCT ACG TGG CAG AAG     288
   Asn Val Thr Ile Leu Tyr Ser Arg Ser Ser Thr Ala Thr Trp Gln Lys
                   85                  90                  95
   AGT GCT GCA TAT GTT TAT CAA GGT GTT TCA AAT TTT ACT TAT TAC AAG          336
   Ser Ala Ala Tyr Val Tyr Gln Gly Val Ser Asn Phe Thr Tyr Tyr Lys
               100                 105                 110
   TTA AAT AAC ACC AAT GGC TTG AAA AGC TAT GAA TTG TGT GAA GAT TAT          384
   Leu Asn Asn Thr Asn Gly Leu Lys Ser Tyr Glu Leu Cys Glu Asp Tyr
           115                 120                 125
   GAA TGC TGC ACT GGC TAT GCT ACC AAC GTA TTT GCC CCG ACA GTG GGC          432
   Glu Cys Cys Thr Gly Tyr Ala Thr Asn Val Phe Ala Pro Thr Val Gly
       130                 135                 140
   GGT TAT ATA CCT GAT GGC TTC AGT TTT AAC AAT TGG TTT ATG CTT ACA          480
   Gly Tyr Ile Pro Asp Gly Phe Ser Phe Asn Asn Trp Phe Met Leu Thr
   145                 150                 155                 160
   AAC AGT TCC ACG TTT GTT AGT GGC AGA TTT GTA ACA AAT CAA CCA TTA          528
   Asn Ser Ser Thr Phe Val Ser Gly Arg Phe Val Thr Asn Gln Pro Leu
                   165                 170                 175
   TTG GTT AAT TGT TTG TGG CCA GTG CCC AGT CTT GGT GTC GCA GCA CAA          576
   Leu Val Asn Cys Leu Trp Pro Val Pro Ser Leu Gly Val Ala Ala Gln
               180                 185                 190
   GAA TTT TGT TTT GAA GGT GCG CAG TTT AGC CAA TGT AAT GGT GTG TCT          624
   Glu Phe Cys Phe Glu Gly Ala Gln Phe Ser Gln Cys Asn Gly Val Ser
           195                 200                 205
   TTA AAC AAT ACA GTG GAT GTC ATT AGA TTC AAC CTT AAT TTT ACC ACA          672
   Leu Asn Asn Thr Val Asp Val Ile Arg Phe Asn Leu Asn Phe Thr Thr
       210                 215                 220
   GAT GTA CAA TCT GGT ATG GGT GCT ACA GTA TTT TCA CTG AAT ACA ACA          720
   Asp Val Gln Ser Gly Met Gly Ala Thr Val Phe Ser Leu Asn Thr Thr
   225                 230                 235                 240
   GGT GGT GTC ATT CTT GAG ATT TCT TGT TAT AAT GAT ACA GTG AGT GAG          768
   Gly Gly Val Ile Leu Glu Ile Ser Cys Tyr Asn Asp Thr Val Ser Glu
                   245                 250                 255
   TCA AGT TTC TAC AGT TAT GGT GAA ATT TCA TTC GGC GTA ACT GAT GGA          816
   Ser Ser Phe Tyr Ser Tyr Gly Glu Ile Ser Phe Gly Val Thr Asp Gly
               260                 265                 270
   CCG CGT TAC TGT TAC GCA CTC TAT AAT GGC ACG GCT CTT AAG TAT TTA          864
   Pro Arg Tyr Cys Tyr Ala Leu Tyr Asn Gly Thr Ala Leu Lys Tyr Leu
           275                 280                 285
   GGA ACA TTA CCA CCT AGT GTC AAG GAA ATT GCT ATT AGT AAG TGG GGC          912
   Gly Thr Leu Pro Pro Ser Val Lys Glu Ile Ala Ile Ser Lys Trp Gly
       290                 295                 300
   CAT TTT TAT ATT AAT GGT TAC AAT TTC TTT AGC ACT TTT CCT ATT GAT          960
   His Phe Tyr Ile Asn Gly Tyr Asn Phe Phe Ser Thr Phe Pro Ile Asp
   305                 310                 315                 320
   TGT ATA TCT TTT AAT TTA ACC ACT GGT GAT AGT GGA GCA TTT TGG ACA         1008
   Cys Ile Ser Phe Asn Leu Thr Thr Gly Asp Ser Gly Ala Phe Trp Thr
                   325                 330                 335
   ATT GCT TAC ACA TCG TAC ACT GAC GCA TTA GTA CAA GTT GAA AAC ACA         1056
   Ile Ala Tyr Thr Ser Tyr Thr Asp Ala Leu Val Gln Val Glu Asn Thr
               340                 345                 350
   GCT ATT AAA AAG GTG ACG TAT TGT AAC AGT CAC ATT AAT AAC ATT AAA      1104
   Ala Ile Lys Lys Val Thr Tyr Cys Asn Ser His Ile Asn Asn Ile Lys
           355                 360                 365
   TGT TCT CAA CTT ACT GCT AAT TTG CAA AAT GGA TTT TAT CCT GTT GCT      1152
   Cys Ser Gln Leu Thr Ala Asn Leu Gln Asn Gly Phe Tyr Pro Val Ala
       370                 375                 380
   TCA AGT GAA GTT GGT CTT GTC AAT AAG AGT GTT GTG TTA CTA CCT AGT      1200
   Ser Ser Glu Val Gly Leu Val Asn Lys Ser Val Val Leu Leu Pro Ser
   385                 390                 395                 400
   TTC TAT TCA CAT ACC AGT GTT AAT ATA ACT ATT GAT CTT GGT ATG AAG      1248
   Phe Tyr Ser His Thr Ser Val Asn Ile Thr Ile Asp Leu Gly Met Lys
                   405                 410                 415
   CGT AGT GGT TAT GGT CAA CCC ATA GCC TCA ACA TTA                      1284
   Arg Ser Gly Tyr Gly Gln Pro Ile Ala Ser Thr Leu
               420                 425
(2) INFORMATION FOR SEQ ID NO:43:
   (i) SEQUENCE CHARACTERISTICS:
      (A) LENGTH: 428 amino acids
      (B) TYPE: amino acid
      (D) TOPOLOGY: linear
   (ii) MOLECULE TYPE: protein
   (xi) SEQUENCE DESCRIPTION: SEQ ID NO:43:
   Arg Pro Leu Leu Lys His Gly Leu Leu Cys Ile Thr Lys Asn Lys Ile
    1               5                  10                  15
   Ile Asp Tyr Asn Thr Phe Thr Ser Ala Gln Trp Ser Ala Ile Cys Leu
               20                  25                  30
   Gly Asp Asp Arg Lys Ile Pro Phe Ser Val Ile Pro Thr Gly Asn Gly
           35                  40                  45
   Thr Lys Ile Phe Gly Leu Glu Trp Asn Asp Asp Tyr Val Thr Ala Tyr
       50                  55                  60
   Ile Ser Asp Arg Ser His His Leu Asn Ile Asn Asn Asn Trp Phe Asn
   65                  70                  75                  80
   Asn Val Thr Ile Leu Tyr Ser Arg Ser Ser Thr Ala Thr Trp Gln Lys
                   85                  90                  95
   Ser Ala Ala Tyr Val Tyr Gln Gly Val Ser Asn Phe Thr Tyr Tyr Lys
               100                 105                 110
   Leu Asn Asn Thr Asn Gly Leu Lys Ser Tyr Glu Leu Cys Glu Asp Tyr
           115                 120                 125
   Glu Cys Cys Thr Gly Tyr Ala Thr Asn Val Phe Ala Pro Thr Val Gly
       130                 135                 140
   Gly Tyr Ile Pro Asp Gly Phe Ser Phe Asn Asn Trp Phe Met Leu Thr
   145                 150                 155                 160
   Asn Ser Ser Thr Phe Val Ser Gly Arg Phe Val Thr Asn Gln Pro Leu
                   165                 170                 175
   Leu Val Asn Cys Leu Trp Pro Val Pro Ser Leu Gly Val Ala Ala Gln
               180                 185                 190
   Glu Phe Cys Phe Glu Gly Ala Gln Phe Ser Gln Cys Asn Gly Val Ser
           195                 200                 205
   Leu Asn Asn Thr Val Asp Val Ile Arg Phe Asn Leu Asn Phe Thr Thr
       210                 215                 220
   Asp Val Gln Ser Gly Met Gly Ala Thr Val Phe Ser Leu Asn Thr Thr
   225                 230                 235                 240
   Gly Gly Val Ile Leu Glu Ile Ser Cys Tyr Asn Asp Thr Val Ser Glu
                   245                 250                 255
   Ser Ser Phe Tyr Ser Tyr Gly Glu Ile Ser Phe Gly Val Thr Asp Gly
               260                 265                 270
   Pro Arg Tyr Cys Tyr Ala Leu Tyr Asn Gly Thr Ala Leu Lys Tyr Leu
           275                 280                 285
   Gly Thr Leu Pro Pro Ser Val Lys Glu Ile Ala Ile Ser Lys Trp Gly
       290                 295                 300
   His Phe Tyr Ile Asn Gly Tyr Asn Phe Phe Ser Thr Phe Pro Ile Asp
   305                 310                 315                 320
   Cys Ile Ser Phe Asn Leu Thr Thr Gly Asp Ser Gly Ala Phe Trp Thr
                   325                 330                 335
   Ile Ala Tyr Thr Ser Tyr Thr Asp Ala Leu Val Gln Val Glu Asn Thr
               340                 345                 350
   Ala Ile Lys Lys Val Thr Tyr Cys Asn Ser His Ile Asn Asn Ile Lys
           355                 360                 365
   Cys Ser Gln Leu Thr Ala Asn Leu Gln Asn Gly Phe Tyr Pro Val Ala
       370                 375                 380
   Ser Ser Glu Val Gly Leu Val Asn Lys Ser Val Val Leu Leu Pro Ser
   385                 390                 395                 400
   Phe Tyr Ser His Thr Ser Val Asn Ile Thr Ile Asp Leu Gly Met Lys
                   405                 410                 415
   Arg Ser Gly Tyr Gly Gln Pro Ile Ala Ser Thr Leu
               420                 425
(2) INFORMATION FOR SEQ ID NO:44:
   (i) SEQUENCE CHARACTERISTICS:
      (A) LENGTH: 546 base pairs
      (B) TYPE: nucleic acid
      (C) STRANDEDNESS: double
      (D) TOPOLOGY: unknown
   (ii) MOLECULE TYPE: DNA (genomic)
   (ix) FEATURE:
      (A) NAME/KEY: CDS
      (B) LOCATION: 1..546
   (xi) SEQUENCE DESCRIPTION: SEQ ID NO:44:
   GAT TGT ATA TCT TTT AAT TTA ACC ACT GGT GAT AGT GGA GCA TTT TGG           48
   Asp Cys Ile Ser Phe Asn Leu Thr Thr Gly Asp Ser Gly Ala Phe Trp
    1               5                  10                  15
   ACA ATT GCT TAC ACA TCG TAC ACT GAC GCA TTA GTA CAA GTT GAA AAC           96
   Thr Ile Ala Tyr Thr Ser Tyr Thr Asp Ala Leu Val Gln Val Glu Asn
               20                  25                  30
   ACA GCT ATT AAA AAG GTG ACG TAT TGT AAC AGT CAC ATT AAT AAC ATT          144
   Thr Ala Ile Lys Lys Val Thr Tyr Cys Asn Ser His Ile Asn Asn Ile
           35                  40                  45
   AAA TGT TCT CAA CTT ACT GCT AAT TTG CAA AAT GGA TTT TAT CCT GTT          192
   Lys Cys Ser Gln Leu Thr Ala Asn Leu Gln Asn Gly Phe Tyr Pro Val
       50                  55                  60
   GCT TCA AGT GAA GTT GGT CTT GTC AAT AAG AGT GTT GTG TTA CTA CCT          240
   Ala Ser Ser Glu Val Gly Leu Val Asn Lys Ser Val Val Leu Leu Pro
   65                  70                  75                  80
   AGT TTC TAT TCA CAT ACC AGT GTT AAT ATA ACT ATT GAT CTT GGT ATG          288
   Ser Phe Tyr Ser His Thr Ser Val Asn Ile Thr Ile Asp Leu Gly Met
                   85                  90                  95
   AAG CGT AGT GGT TAT GGT CAA CCC ATA GCC TCA ACA TTA AGT AAC ATC          336
   Lys Arg Ser Gly Tyr Gly Gln Pro Ile Ala Ser Thr Leu Ser Asn Ile
               100                 105                 110
   ACA CTA CCA ATG CAG GAT AAT AAC ACC GAT GTG TAC TGC ATT CGT TCT          384
   Thr Leu Pro Met Gln Asp Asn Asn Thr Asp Val Tyr Cys Ile Arg Ser
           115                 120                 125
   AAC CAA TTT TCA GTT TAC GTT CAT TCC ACT TGT AAA AGT TCT TTA TGG          432
   Asn Gln Phe Ser Val Tyr Val His Ser Thr Cys Lys Ser Ser Leu Trp
       130                 135                 140
   GAC GAT GTG TTT AAT TCC GAC TGC ACA GAT GTT TTA TAT GCT ACA GCT          480
   Asp Asp Val Phe Asn Ser Asp Cys Thr Asp Val Leu Tyr Ala Thr Ala
   145                 150                 155                 160
   GTT ATA AAA ACT GGT ACT TGT CCT TTC TCG TTT GAT AAA TTG AAC AAT          528
   Val Ile Lys Thr Gly Thr Cys Pro Phe Ser Phe Asp Lys Leu Asn Asn
                   165                 170                 175
   TAC TTA ACT TTT AAC AAG                                                  546
   Tyr Leu Thr Phe Asn Lys
               180
(2) INFORMATION FOR SEQ ID NO:45:
   (i) SEQUENCE CHARACTERISTICS:
      (A) LENGTH: 182 amino acids
      (B) TYPE: amino acid
      (D) TOPOLOGY: linear
   (ii) MOLECULE TYPE: protein
   (xi) SEQUENCE DESCRIPTION: SEQ ID NO:45:
   Asp Cys Ile Ser Phe Asn Leu Thr Thr Gly Asp Ser Gly Ala Phe Trp
    1               5                  10                  15
   Thr Ile Ala Tyr Thr Ser Tyr Thr Asp Ala Leu Val Gln Val Glu Asn
               20                  25                  30
   Thr Ala Ile Lys Lys Val Thr Tyr Cys Asn Ser His Ile Asn Asn Ile
           35                  40                  45
   Lys Cys Ser Gln Leu Thr Ala Asn Leu Gln Asn Gly Phe Tyr Pro Val
       50                  55                  60
   Ala Ser Ser Glu Val Gly Leu Val Asn Lys Ser Val Val Leu Leu Pro
   65                  70                  75                  80
   Ser Phe Tyr Ser His Thr Ser Val Asn Ile Thr Ile Asp Leu Gly Met
                   85                  90                  95
   Lys Arg Ser Gly Tyr Gly Gln Pro Ile Ala Ser Thr Leu Ser Asn Ile
               100                 105                 110
   Thr Leu Pro Met Gln Asp Asn Asn Thr Asp Val Tyr Cys Ile Arg Ser
           115                 120                 125
   Asn Gln Phe Ser Val Tyr Val His Ser Thr Cys Lys Ser Ser Leu Trp
       130                 135                 140
   Asp Asp Val Phe Asn Ser Asp Cys Thr Asp Val Leu Tyr Ala Thr Ala
   145                 150                 155                 160
   Val Ile Lys Thr Gly Thr Cys Pro Phe Ser Phe Asp Lys Leu Asn Asn
                   165                 170                 175
   Tyr Leu Thr Phe Asn Lys
               180
(2) INFORMATION FOR SEQ ID NO:46:
   (i) SEQUENCE CHARACTERISTICS:
      (A) LENGTH: 38 base pairs
      (B) TYPE: nucleic acid
      (C) STRANDEDNESS: single
      (D) TOPOLOGY: unknown
   (ii) MOLECULE TYPE: DNA (genomic)
   (xi) SEQUENCE DESCRIPTION: SEQ ID NO:46:
   TAAATAGGCC TTTAGTGGAC ATGCACTTTT TCAATTGG                               38
(2) INFORMATION FOR SEQ ID NO:47:
   (i) SEQUENCE CHARACTERISTICS:
      (A) LENGTH: 39 base pairs
      (B) TYPE: nucleic acid
      (C) STRANDEDNESS: single
      (D) TOPOLOGY: unknown
   (ii) MOLECULE TYPE: DNA (genomic)
   (xi) SEQUENCE DESCRIPTION: SEQ ID NO:47:
   TTAGTAGGCC TGTCGAGGCT ATGGGTTGAC CATAACCAC 39
(2) INFORMATION FOR SEQ ID NO:48:
   (i) SEQUENCE CHARACTERISTICS:
      (A) LENGTH: 37 base pairs
      (B) TYPE: nucleic acid
      (C) STRANDEDNESS: single
      (D) TOPOLOGY: unknown
   (ii) MOLECULE TYPE: DNA (genomic)
   (xi) SEQUENCE DESCRIPTION: SEQ ID NO:48:
   CAGATCCCGG GTGTACAATC TGGTATGGGT GCTACAG 37
(2) INFORMATION FOR SEQ ID NO:49:
   (i) SEQUENCE CHARACTERISTICS:
      (A) LENGTH: 39 base pairs
      (B) TYPE: nucleic acid
      (C) STRANDEDNESS: single
      (D) TOPOLOGY: unknown
   (ii) MOLECULE TYPE: DNA (genomic)
   (xi) SEQUENCE DESCRIPTION: SEQ ID NO:49:
   GTGCCCCCGG GTATGATTGT GCTCGTAACT TGCCTCTTG 39
(2) INFORMATION FOR SEQ ID NO:50:
   (i) SEQUENCE CHARACTERISTICS:
      (A) LENGTH: 43 base pairs
      (B) TYPE: nucleic acid
      (C) STRANDEDNESS: single
      (D) TOPOLOGY: unknown
   (ii) MOLECULE TYPE: DNA (genomic)
   (xi) SEQUENCE DESCRIPTION: SEQ ID NO:50:
   AGCACCCATA CCAGATTGTA CATCTGCAGT GAAATTAAGA TTG 43
(2) INFORMATION FOR SEQ ID NO:51:
   (i) SEQUENCE CHARACTERISTICS:
      (A) LENGTH: 128 amino acids
      (B) TYPE: amino acid
      (D) TOPOLOGY: unknown
   (ii) MOLECULE TYPE: protein
   (xi) SEQUENCE DESCRIPTION: SEQ ID NO:51:
   Met Ile Val Leu Val Thr Cys Leu Leu Phe Ser Tyr Asn Ser Val Ile
   1               5                   10                  15
   Cys Thr Ser Asn Asn Asp Cys Val Gln Val Asn Val Thr Gln Leu Pro
               20                  25                  30
   Gly Asn Glu Asn Ile Ile Lys Asp Phe Leu Phe His Thr Phe Lys Glu
           35                  40                  45
   Glu Gly Ser Val Val Val Gly Gly Tyr Tyr Pro Thr Glu Val Trp Tyr
       50                  55                  60
   Asn Cys Ser Arg Ser Ala Thr Thr Thr Ala Tyr Lys Asp Phe Ser Asn
   65                  70                  75                  80
   Ile His Ala Phe Tyr Phe Asp Met Glu Ala Met Glu Asn Ser Thr Gly
                   85                  90                  95
   Asn Ala Arg Gly Lys Pro Leu Leu Val His Val His Gly Asp Pro Val
               100                 105                 110
   Ser Ile Ile Ile Tyr Ile Ser Ala Tyr Arg Asp Asp Val Gln Gly Arg
           115                 120                 125
(2) INFORMATION FOR SEQ ID NO:52:
   (i) SEQUENCE CHARACTERISTICS:
      (A) LENGTH: 1101 amino acids
      (B) TYPE: amino acid
      (D) TOPOLOGY: unknown
   (ii) MOLECULE TYPE: protein
   (xi) SEQUENCE DESCRIPTION: SEQ ID NO:52:
   Asp Val Gln Ser Gly Met Gly Ala Thr Val Phe Ser Leu Asn Thr Thr
   1               5                   10                  15
   Gly Gly Val Ile Leu Glu Ile Ser Cys Tyr Asn Asp Thr Val Ser Glu
               20                  25                  30
   Ser Ser Phe Tyr Ser Tyr Gly Glu Ile Ser Phe Gly Val Thr Asp Gly
           35                  40                  45
   Pro Arg Tyr Cys Tyr Ala Leu Tyr Asn Gly Thr Ala Leu Lys Tyr Leu
       50                  55                  60
   Gly Thr Leu Pro Pro Ser Val Lys Glu Ile Ala Ile Ser Lys Trp Gly
   65                  70                  75                  80
   His Phe Tyr Ile Asn Gly Tyr Asn Phe Phe Ser Thr Phe Pro Ile Asp
                   85                  90                  95
   Cys Ile Ser Phe Asn Leu Thr Thr Gly Asp Ser Gly Ala Phe Trp Thr
               100                 105                 110
   Ile Ala Tyr Thr Ser Tyr Thr Asp Ala Leu Val Gln Val Glu Asn Thr
           115                 120                 125
   Ala Ile Lys Lys Val Thr Tyr Cys Asn Ser His Ile Asn Asn Ile Lys
       130                 135                 140
   Cys Ser Gln Leu Thr Ala Asn Leu Gln Asn Gly Phe Tyr Pro Val Ala
   145                 150                 155                 160
   Ser Ser Glu Val Gly Leu Val Asn Lys Ser Val Val Leu Leu Pro Ser
                   165                 170                 175
   Phe Tyr Ser His Thr Ser Val Asn Ile Thr Ile Asp Leu Gly Met Lys
               180                 185                 190
   Arg Ser Gly Tyr Gly Gln Pro Ile Ala Ser Thr Leu Ser Asn Ile Thr
           195                 200                 205
   Leu Pro Met Gln Asp Asn Asn Thr Asp Val Tyr Cys Ile Arg Ser Asn
       210                 215                 220
   Gln Phe Ser Val Tyr Val His Ser Thr Cys Lys Ser Ser Leu Trp Asp
   225                 230                 235                 240
   Asp Val Phe Asn Ser Asp Cys Thr Asp Val Leu Tyr Ala Thr Ala Val
                   245                 250                 255
   Ile Lys Thr Gly Thr Cys Pro Phe Ser Phe Asp Lys Leu Asn Asn Tyr
               260                 265                 270
   Leu Thr Phe Asn Lys Phe Cys Leu Ser Leu Asn Pro Val Gly Ala Asn
           275                 280                 285
   Cys Lys Phe Asp Val Ala Ala Arg Thr Arg Thr Asn Glu Gln Val Val
       290                 295                 300
   Arg Ser Leu Tyr Val Ile Tyr Glu Glu Gly Asp Asn Ile Val Gly Val
   305                 310                 315                 320
   Pro Ser Asp Asn Ser Gly Leu His Asp Leu Ser Val Leu His Leu Asp
                   325                 330                 335
   Ser Cys Thr Asp Tyr Asn Ile Tyr Gly Arg Thr Gly Val Gly Ile Ile
               340                 345                 350
   Arg Gln Thr Asn Ser Thr Leu Leu Ser Gly Leu Tyr Tyr Thr Ser Leu
           355                 360                 365
   Ser Gly Asp Leu Leu Gly Phe Lys Asn Val Ser Asp Gly Val Ile Tyr
       370                 375                 380
   Ser Val Thr Pro Cys Asp Val Ser Ala Gln Ala Ala Val Ile Asp Gly
   385                 390                 395                 400
   Ala Ile Val Gly Ala Met Thr Ser Ile Asn Ser Glu Met Leu Gly Leu
                   405                 410                 415
   Thr His Trp Thr Thr Thr Pro Asn Phe Tyr Tyr Tyr Ser Ile Tyr Asn
               420                 425                 430
   Tyr Thr Asn Glu Arg Thr Arg Gly Thr Ala Ile Asp Ser Asn Asp Val
           435                 440                 445
   Asp Cys Glu Pro Ile Ile Thr Tyr Ser Asn Ile Gly Val Cys Lys Asn
       450                 455                 460
   Gly Ala Leu Val Phe Ile Asn Val Thr His Ser Asp Gly Asp Val Gln
   465                 470                 475                 480
   Pro Ile Ser Thr Gly Asn Val Thr Ile Pro Thr Asn Phe Thr Ile Ser
                   485                 490                 495
   Val Gln Val Glu Tyr Ile Gln Val Tyr Thr Thr Pro Val Ser Ile Asp
               500                 505                 510
   Cys Ser Arg Tyr Val Cys Asn Gly Asn Pro Arg Cys Asn Lys Leu Leu
           515                 520                 525
   Thr Gln Tyr Val Ser Ala Cys Gln Thr Ile Glu Gln Ala Leu Ala Met
       530                 535                 540
   Gly Ala Arg Leu Glu Asn Met Glu Ile Asp Ser Met Leu Phe Val Ser
   545                 550                 555                 560
   Glu Asn Ala Leu Lys Leu Ala Ser Val Glu Ala Phe Asn Ser Thr Glu
                   565                 570                 575
   Thr Leu Asp Pro Ile Tyr Lys Glu Trp Pro Asn Ile Gly Gly Ser Trp
               580                 585                 590
   Leu Gly Gly Leu Lys Asp Ile Leu Pro Ser His Asn Ser Lys Arg Lys
           595                 600                 605
   Tyr Arg Ser Ala Ile Glu Asp Leu Leu Phe Asp Lys Val Val Thr Ser
       610                 615                 620
   Gly Leu Gly Thr Val Asp Glu Asp Tyr Lys Arg Cys Thr Gly Gly Tyr
   625                 630                 635                 640
   Asp Ile Ala Asp Leu Val Cys Ala Gln Tyr Tyr Asn Gly Ile Met Val
                   645                 650                 655
   Leu Pro Gly Val Ala Asn Asp Asp Lys Met Ala Met Tyr Thr Ala Ser
               660                 665                 670
   Leu Ala Gly Gly Ile Thr Leu Gly Ala Leu Gly Gly Gly Ala Val Ser
           675                 680                 685
   Ile Pro Phe Ala Ile Ala Val Gln Ala Arg Leu Asn Tyr Val Ala Leu
       690                 695                 700
   Gln Thr Asp Val Leu Ser Lys Asn Gln Gln Ile Leu Ala Asn Ala Phe
   705                 710                 715                 720
   Asn Gln Ala Ile Gly Asn Ile Thr Gln Ala Phe Gly Lys Val Asn Asp
                   725                 730                 735
   Ala Ile His Gln Thr Ser Gln Gly Leu Ala Thr Val Ala Lys Ala Leu
               740                 745                 750
   Ala Lys Val Gln Asp Val Val Asn Thr Gln Gly Gln Ala Leu Ser His
           755                 760                 765
   Leu Thr Val Gln Leu Gln Asn Asn Phe Gln Ala Ile Ser Ser Ser Ile
       770                 775                 780
   Ser Asp Ile Tyr Asn Arg Leu Asp Glu Leu Ser Ala Asp Ala Gln Val
   785                 790                 795                 800
   Asp Arg Leu Ile Thr Gly Arg Leu Thr Ala Leu Asn Ala Phe Val Ser
                   805                 810                 815
   Gln Thr Leu Thr Arg Gln Ala Glu Val Arg Ala Ser Arg Gln Leu Ala
               820                 825                 830
   Lys Asp Lys Val Asn Glu Cys Val Arg Ser Gln Ser Gln Arg Phe Gly
           835                 840                 845
   Phe Cys Gly Asn Gly Thr His Leu Phe Ser Leu Ala Asn Ala Ala Pro
       850                 855                 860
   Asn Gly Met Ile Phe Phe His Thr Val Leu Leu Pro Thr Ala Tyr Glu
   865                 870                 875                 880
   Thr Val Thr Ala Trp Ser Gly Ile Cys Ala Ser Asp Gly Asp Arg Thr
                   885                 890                 895
   Phe Gly Leu Val Val Lys Asp Val Gln Leu Thr Leu Phe Arg Asn Leu
               900                 905                 910
   Asp Asp Lys Phe Tyr Leu Thr Pro Arg Thr Met Tyr Gln Pro Arg Val
           915                 920                 925
   Ala Thr Ser Ser Asp Phe Val Gln Ile Glu Gly Cys Asp Val Leu Phe
       930                 935                 940
   Val Asn Ala Thr Val Ile Asp Leu Pro Ser Ile Ile Pro Asp Tyr Ile
   945                 950                 955                 960
   Asp Ile Asn Gln Thr Val Gln Asp Ile Leu Glu Asn Phe Arg Pro Asn
                   965                 970                 975
   Trp Thr Val Pro Glu Leu Pro Leu Asp Ile Phe Asn Ala Thr Tyr Leu
               980                 985                 990
   Asn Leu Thr Gly Glu Ile Asn Asp Leu Glu Phe Arg Ser Glu Lys Leu
           995                 1000                1005
   His Asn Thr Thr Val Glu Leu Ala Ile Leu Ile Asp Asn Ile Asn Asn
       1010                1015                1020
   Thr Leu Val Asn Leu Glu Trp Leu Asn Arg Ile Glu Thr Tyr Val Lys
   1025                1030                1035                1040
   Trp Pro Trp Tyr Val Trp Leu Leu Ile Gly Leu Val Val Ile Phe Cys
                   1045                1050                1055
   Ile Pro Ile Leu Leu Phe Cys Cys Cys Ser Thr Gly Cys Cys Gly Cys
               1060                1065                1070
   Ile Gly Cys Leu Gly Ser Cys Cys His Ser Ile Cys Ser Arg Arg Arg
           1075                1080                1085
   Phe Glu Ser Tyr Glu Pro Ile Glu Lys Val His Val His
       1090                1095                1100
(2) INFORMATION FOR SEQ ID NO:53:
   (i) SEQUENCE CHARACTERISTICS:
      (A) LENGTH: 362 amino acids
      (B) TYPE: amino acid
      (D) TOPOLOGY: unknown
   (ii) MOLECULE TYPE: protein
   (xi) SEQUENCE DESCRIPTION: SEQ ID NO:53:
   Met Ile Val Leu Val Thr Cys Leu Leu Phe Ser Tyr Asn Ser Val Ile
   1               5                   10                  15
   Cys Thr Ser Asn Asn Asp Cys Val Gln Val Asn Val Thr Gln Leu Pro
               20                  25                  30
   Gly Asn Glu Asn Ile Ile Lys Asp Phe Leu Phe His Thr Phe Lys Glu
           35                  40                  45
   Glu Gly Ser Val Val Val Gly Gly Tyr Tyr Pro Thr Glu Val Trp Tyr
       50                  55                  60
   Asn Cys Ser Arg Ser Ala Thr Thr Thr Ala Tyr Lys Asp Phe Ser Asn
   65                  70                  75                  80
   Ile His Ala Phe Tyr Phe Asp Met Glu Ala Met Glu Asn Ser Thr Gly
                   85                  90                  95
   Asn Ala Arg Gly Lys Pro Leu Leu Val His Val His Gly Asp Pro Val
               100                 105                 110
   Ser Ile Ile Ile Tyr Ile Ser Ala Tyr Arg Asp Asp Val Gln Gly Arg
           115                 120                 125
   Pro Leu Leu Lys His Gly Leu Leu Cys Ile Thr Lys Asn Lys Ile Ile
       130                 135                 140
   Asp Tyr Asn Thr Phe Thr Ser Ala Gln Trp Ser Ala Ile Cys Leu Gly
   145                 150                 155                 160
   Asp Asp Arg Lys Ile Pro Phe Ser Val Ile Pro Thr Gly Asn Gly Thr
                   165                 170                 175
   Lys Ile Phe Gly Leu Glu Trp Asn Asp Asp Tyr Val Thr Ala Tyr Ile
               180                 185                 190
   Ser Asp Arg Ser His His Leu Asn Ile Asn Asn Asn Trp Phe Asn Asn
           195                 200                 205
   Val Thr Ile Leu Tyr Ser Arg Ser Ser Thr Ala Thr Trp Gln Lys Ser
       210                 215                 220
   Ala Ala Tyr Val Tyr Gln Gly Val Ser Asn Phe Thr Tyr Tyr Lys Leu
   225                 230                 235                 240
   Asn Asn Thr Asn Gly Leu Lys Ser Tyr Glu Leu Cys Glu Asp Tyr Glu
                   245                 250                 255
   Cys Cys Thr Gly Tyr Ala Thr Asn Val Phe Ala Pro Thr Val Gly Gly
               260                 265                 270
   Tyr Ile Pro Asp Gly Phe Ser Phe Asn Asn Trp Phe Met Leu Thr Asn
           275                 280                 285
   Ser Ser Thr Phe Val Ser Gly Arg Phe Val Thr Asn Gln Pro Leu Leu
       290                 295                 300
   Val Asn Cys Leu Trp Pro Val Pro Ser Leu Gly Val Ala Ala Gln Glu
   305                 310                 315                 320
   Phe Cys Phe Glu Gly Ala Gln Phe Ser Gln Cys Asn Gly Val Ser Leu
                   325                 330                 335
   Asn Asn Thr Val Asp Val Ile Arg Phe Asn Leu Asn Phe Thr Thr Asp
               340                 345                 350
   Val Gln Ser Gly Met Gly Ala Thr Val Phe
           355                 360
(2) INFORMATION FOR SEQ ID NO:54:
   (i) SEQUENCE CHARACTERISTICS:
      (A) LENGTH: 1101 amino acids
      (B) TYPE: amino acid
      (D) TOPOLOGY: unknown
   (ii) MOLECULE TYPE: protein
   (xi) SEQUENCE DESCRIPTION: SEQ ID NO:54:
   Ala Ala Tyr Val Tyr Gln Gly Val Ser Asn Phe Thr Tyr Tyr Lys Leu
   1               5                   10                  15
   Asn Asn Thr Asn Gly Leu Lys Ser Tyr Glu Leu Cys Glu Asp Tyr Glu
               20                  25                  30
   Cys Cys Thr Gly Tyr Ala Thr Asn Val Phe Ala Pro Thr Val Gly Gly
           35                  40                  45
   Tyr Ile Pro Asp Gly Phe Ser Phe Asn Asn Trp Phe Met Leu Thr Asn
       50                  55                  60
   Ser Ser Thr Phe Val Ser Gly Arg Phe Val Thr Asn Gln Pro Leu Leu
   65                  70                  75                  80
   Val Asn Cys Leu Trp Pro Val Pro Ser Leu Gly Val Ala Ala Gln Glu
                   85                  90                  95
   Phe Cys Phe Glu Gly Ala Gln Phe Ser Gln Cys Asn Gly Val Ser Leu
               100                 105                 110
   Asn Asn Thr Val Asp Val Ile Arg Phe Asn Leu Asn Phe Thr Thr Asp
           115                 120                 125
   Val Gln Ser Gly Met Gly Ala Thr Val Phe Ser Leu Asn Thr Thr Gly
       130                 135                 140
   Gly Val Ile Leu Glu Ile Ser Cys Tyr Asn Asp Thr Val Ser Glu Ser
   145                 150                 155                 160
   Ser Phe Tyr Ser Tyr Gly Glu Ile Ser Phe Gly Val Thr Asp Gly Pro
                   165                 170                 175
   Arg Tyr Cys Tyr Ala Leu Tyr Asn Gly Thr Ala Leu Lys Tyr Leu Gly
               180                 185                 190
   Thr Leu Pro Pro Ser Val Lys Glu Ile Ala Ile Ser Lys Trp Gly His
           195                 200                 205
   Phe Tyr Ile Asn Gly Tyr Asn Phe Phe Ser Thr Phe Pro Ile Asp Cys
       210                 215                 220
   Ile Ser Phe Asn Leu Thr Thr Gly Asp Ser Gly Ala Phe Trp Thr Ile
   225 230 235 240
   Ala Tyr Thr Ser Tyr Thr Asp Ala Leu Val Gln Val Glu Asn Thr Ala
   245 250 255
   Ile Lys Lys Val Thr Tyr Cys Asn Ser His Ile Asn Asn Ile Lys Cys
   260 265 270
   Ser Gln Leu Thr Ala Asn Leu Gln Asn Gly Phe Tyr Pro Val Ala Ser
   275 280 285
   Ser Glu Val Gly Leu Val Asn Lys Ser Val Val Leu Leu Pro Ser Phe
   290 295 300
   Tyr Ser His Thr Ser Val Asn Ile Thr Ile Asp Leu Gly Met Lys Arg
   305 310 315 320
   Ser Gly Tyr Gly Gln Pro Ile Ala Ser Thr Leu Ser Asn Ile Thr Leu
   325 330 335
   Pro Met Gln Asp Asn Asn Thr Asp Val Tyr Cys Ile Arg Ser Asn Gln
   340 345 350
   Phe Ser Val Tyr Val His Ser Thr Cys Lys Ser Ser Leu Trp Asp Asp
   355 360 365
   Val Phe Asn Ser Asp Cys Thr Asp Val Leu Tyr Ala Thr Ala Val Ile
   370 375 380
   Lys Thr Gly Thr Cys Pro Phe Ser Phe Asp Lys Leu Asn Asn Tyr Leu
   385 390 395 400
   Thr Phe Asn Lys Phe Cys Leu Ser Leu Asn Pro Val Gly Ala Asn Cys
   405 410 415
   Lys Phe Asp Val Ala Ala Arg Thr Arg Thr Asn Glu Gln Val Val Arg
   420 425 430
   Ser Leu Tyr Val Ile Tyr Glu Glu Gly Asp Asn Ile Val Gly Val Pro
   435 440 445
   Ser Asp Asn Ser Gly Leu His Asp Leu Ser Val Leu His Leu Asp Ser
   450 455 460
   Cys Thr Asp Tyr Asn Ile Tyr Gly Arg Thr Gly Val Gly Ile Ile Arg
   465 470 475 480
   Gln Thr Asn Ser Thr Leu Leu Ser Gly Leu Tyr Tyr Thr Ser Leu Ser
   485 490 495
   Gly Asp Leu Leu Gly Phe Lys Asn Val Ser Asp Gly Val Ile Tyr Ser
   500 505 510
   Val Thr Pro Cys Asp Val Ser Ala Gln Ala Ala Val Ile Asp Gly Ala
   515 520 525
   Ile Val Gly Ala Met Thr Ser Ile Asn Ser Glu Met Leu Gly Leu Thr
   530 535 540
   His Trp Thr Thr Thr Pro Asn Phe Tyr Tyr Tyr Ser Ile Tyr Asn Tyr
   545 550 555 560
   Thr Asn Glu Arg Thr Arg Gly Thr Ala Ile Asp Ser Asn Asp Val Asp
   565 570 575
   Cys Glu Pro Ile Ile Thr Tyr Ser Asn Ile Gly Val Cys Lys Asn Gly
   580 585 590
   Ala Leu Val Phe Ile Asn Val Thr His Ser Asp Gly Asp Val Gln Pro
   595 600 605
   Ile Ser Thr Gly Asn Val Thr Ile Pro Thr Asn Phe Thr Ile Ser Val
   610 615 620
   Gln Val Glu Tyr Ile Gln Val Tyr Thr Thr Pro Val Ser Ile Asp Cys
   625 630 635 640
   Ser Arg Tyr Val Cys Asn Gly Asn Pro Arg Cys Asn Lys Leu Leu Thr
   645 650 655
   Gln Tyr Val Ser Ala Cys Gln Thr Ile Glu Gln Ala Leu Ala Met Gly
   660 665 670
   Ala Arg Leu Glu Asn Met Glu Ile Asp Ser Met Leu Phe Val Ser Glu
   675 680 685
   Asn Ala Leu Lys Leu Ala Ser Val Glu Ala Phe Asn Ser Thr Glu Thr
   690 695 700
   Leu Asp Pro Ile Tyr Lys Glu Trp Pro Asn Ile Gly Gly Ser Trp Leu
   705 710 715 720
   Gly Gly Leu Lys Asp Ile Leu Pro Ser His Asn Ser Lys Arg Lys Tyr
   725 730 735
   Arg Ser Ala Ile Glu Asp Leu Leu Phe Asp Lys Val Val Thr Ser Gly
   740 745 750
   Leu Gly Thr Val Asp Glu Asp Tyr Lys Arg Cys Thr Gly Gly Tyr Asp
   755 760 765
   Ile Ala Asp Leu Val Cys Ala Gln Tyr Tyr Asn Gly Ile Met Val Leu
   770 775 780
   Pro Gly Val Ala Asn Asp Asp Lys Met Ala Met Tyr Thr Ala Ser Leu
   785 790 795 800
   Ala Gly Gly Ile Thr Leu Gly Ala Leu Gly Gly Gly Ala Val Ser Ile
   805 810 815
   Pro Phe Ala Ile Ala Val Gln Ala Arg Leu Asn Tyr Val Ala Leu Gln
   820 825 830
   Thr Asp Val Leu Ser Lys Asn Gln Gln Ile Leu Ala Asn Ala Phe Asn
   835 840 845
   Gln Ala Ile Gly Asn Ile Thr Gln Ala Phe Gly Lys Val Asn Asp Ala
   850 855 860
   Ile His Gln Thr Ser Gln Gly Leu Ala Thr Val Ala Lys Ala Leu Ala
   865 870 875 880
   Lys Val Gln Asp Val Val Asn Thr Gln Gly Gln Ala Leu Ser His Leu
   885 890 895
   Thr Val Gln Leu Gln Asn Asn Phe Gln Ala Ile Ser Ser Ser Ile Ser
               900                 905                 910
   Asp Ile Tyr Asn Arg Leu Asp Glu Leu Ser Ala Asp Ala Gln Val Asp
           915                 920                 925
   Arg Leu Ile Thr Gly Arg Leu Thr Ala Leu Asn Ala Phe Val Ser Gln
       930                 935                 940
   Thr Leu Thr Arg Gln Ala Glu Val Arg Ala Ser Arg Gln Leu Ala Lys
   945                 950                 955                 960
   Asp Lys Val Asn Glu Cys Val Arg Ser Gln Ser Gln Arg Phe Gly Phe
                   965                 970                 975
   Cys Gly Asn Gly Thr His Leu Phe Ser Leu Ala Asn Ala Ala Pro Asn
               980                 985                 990
   Gly Met Ile Phe Phe His Thr Val Leu Leu Pro Thr Ala Tyr Glu Thr
           995                 1000                1005
   Val Thr Ala Trp Ser Gly Ile Cys Ala Ser Asp Gly Asp Arg Thr Phe
       1010                1015                1020
   Gly Leu Val Val Lys Asp Val Gln Leu Thr Leu Phe Arg Asn Leu Asp
   1025                1030                1035                1040
   Asp Lys Phe Tyr Leu Thr Pro Arg Thr Met Tyr Gln Pro Arg Val Ala
                   1045                1050                1055
   Thr Ser Ser Asp Phe Val Gln Ile Glu Gly Cys Asp Val Leu Phe Val
               1060                1065                1070
   Asn Ala Thr Val Ile Asp Leu Pro Ser Ile Ile Pro Asp Tyr Ile Asp
           1075                1080                1085
   Ile Asn Gln Thr Val Gln Asp Ile Leu Glu Asn Phe Arg
       1090                1095                1100
(2) INFORMATION FOR SEQ ID NO:55:
   (i) SEQUENCE CHARACTERISTICS:
      (A) LENGTH: 701 base pairs
      (B) TYPE: nucleic acid
      (C) STRANDEDNESS: double
      (D) TOPOLOGY: unknown
   (ii) MOLECULE TYPE: DNA (genomic)
   (xi) SEQUENCE DESCRIPTION: SEQ ID NO:55:
   TCAACCATTA TTGGTTAATT GTTTGTGGCC AGTGCCCAGT CTTGGTGTCG CAGCACAAGA      60
   ATTTTGTTTT GAAGGTGCGC AGTTTAGCCA ATGTAATGGT GTGTCTTTAA ACAATACAGT     120
   GGATGTCATT AGATTCAACC TTAATTTTAC CACAGATGTA CAATCTGGTA TGGGTGCTAC     180
   AGTATTTTCA CTGAATACAA CAGGTGGTGT CATTCTTGAG ATTTCTTGTT ATAATGATAC     240
   AGTGAGTGAG TCAAGTTTCT ACAGTTATGG TGAAATTTCA TTCGGCGTAA CTGATGGACC     300
   GCGTTACTGT TACGCACTCT ATAATGGCAC GGCTCTTAAG TATTTAGGAA CATTACCACC     360
   TAGTGTCAAG GAAATTGCTA TTAGTAAGTG GGGCCATTTT TATATTAATG GTTACAATTT     420
   CTTTAGCACT TTTCCTATTG ATTGTATATC TTTTAATTTA ACCACTGGTG ATAGTGGAGC     480
   ATTTTGGACA ATTGCTTACA CATCGTACAC TGACGCATTA GTACAAGTTG AAAACACAGC     540
   TATTAAAAAG GTGACGTATT GTAACAGTCA CATTAATAAC ATTAAATGTT CTCAACTTAC     600
   TGCTAATTTG CAAAATGGAT TTTATCCTGT TGCTTCAAGT GAAGTTGGTC TTGTCAATAA     660
   GAGTGTTGTG TTACTACCTA GTTTCTATTC ACATACCAGT G                         701
(2) INFORMATION FOR SEQ ID NO:56:
   (i) SEQUENCE CHARACTERISTICS:
      (A) LENGTH: 1401 base pairs
      (B) TYPE: nucleic acid
      (C) STRANDEDNESS: double
      (D) TOPOLOGY: unknown
   (ii) MOLECULE TYPE: DNA (genomic)
   (xi) SEQUENCE DESCRIPTION: SEQ ID NO:56:
   AGCACCGGTA ATGTCACGAT ACCTACAAAT TTTACCATAT CTGTGCAAGT TGAGTACATT      60
   CAGGTTTACA CTACACCGGT GTCAATAGAT TGTTCAAGGT ACGTTTGCAA TGGTAACCCT     120
   AGATGCAATA AATTGTTAAC GCAATACGTT TCTGCATGTC AAACTATTGA GCAAGCACTT     180
   GCAATGGGTG CCAGACTTGA AAACATGGAG ATTGATTCCA TGTTGTTTGT TTCGGAAAAT     240
   GCCCTTAAAT TGGCATCTGT TGAAGCATTC AATAGTACGG AAACTTTAGA TCCTATTTAC     300
   AAAGAATGGC CTAACATTGG TGGTTCTTGG CTAGGAGGTT TAAAAGACAT ATTGCCATCT     360
   CACAACAGCA AACGTAAGTA CCGGTCGGCT ATAGAAGATT TGCTTTTTGA TAAGGTTGTA     420
   ACATCTGGCT TAGGTACAGT TGATGAAGAT TATAAACGTT GTACAGGTGG TTATGACATA     480
   GCTGACTTAG TGTGTGCACA ATATTACAAT GGCATCATGG TGCTACCTGG TGTAGCTAAT     540
   GATGACAAGA TGGCTATGTA CACTGCATCT CTTGCAGGTG GTATAACATT AGGTGCACTT     600
   GGTGGTGGCG CAGTGTCTAT ACCTTTTGCA ATAGCAGTTC AAGCCAGACT TAATTATGTT     660
   GCTCTACAAA CTGATGTATT GAGCAAGAAC CAGCAGATCC TGGCTAATGC TTTCAATCAA     720
   GCTATTGGTA ACATTACACA GGCATTTGGT AAGGTTAATG ATGCTATACA TCAAACGTCA     780
   CAAGGTCTTG CTACTGTTGC TAAAGCATTG GCAAAAGTGC AAGATGTTGT TAACACACAA     840
   GGGCAAGCTT TAAGCCACCT AACAGTACAA TTGCAAAATA ATTTCCAAGC CATTAGTAGT     900
   TCCATTAGTG ACATTTATAA CAGGCTTGAT GAATTGAGTG CTGATGCACA AGTTGACAGG     960
   CTGATTACAG GAAGACTTAC AGCACTTAAT GCATTTGTGT CTCAGACTTT AACCAGACAA    1020
   GCAGAGGTTA GGGCTAGCAG ACAGCTTGCT AAAGACAAGG TAAATGAATG CGTTAGGTCT    1080
   CAATCTCAGA GATTTGGATT CTGTGGTAAT GGTACACATT TATTTTCACT TGCAAATGCA    1140
   GCACCAAATG GCATGATCTT CTTTCACACA GTGCTATTAC CAACAGCTTA TGAAACCGTG    1200
   ACGGCCTGGT CAGGTATTTG TGCATCAGAT GGCGATCGTA CTTTTGGACT TGTTGTTAAG    1260
   GATGTCCAGT TGACGCTGTT TCGCAATCTA GATGACAAAT TCTATTTGAC TCCCAGAACT    1320
   ATGTATCAGC CTAGAGTTGC AACTAGTTCT GATTTTGTTC AAATTGAAGG ATGTGATGTG    1380
   TTGTTTGTTA ATGCAACTGT A                                              1401
(2) INFORMATION FOR SEQ ID NO:57:
   (i) SEQUENCE CHARACTERISTICS:
      (A) LENGTH: 250 amino acids
      (B) TYPE: amino acid
      (D) TOPOLOGY: unknown
   (ii) MOLECULE TYPE: protein
   (xi) SEQUENCE DESCRIPTION: SEQ ID NO:57:
   Met Ile Val Leu Val Thr Cys Leu Leu Phe Ser Tyr Asn Ser Val Ile
   1               5                   10                  15
   Cys Thr Ser Asn Asn Asp Cys Val Gln Val Asn Val Thr Gln Leu Pro
               20                  25                  30
   Gly Asn Glu Asn Ile Ile Lys Asp Phe Leu Phe His Thr Phe Lys Glu
           35                  40                  45
   Glu Gly Ser Val Val Val Gly Gly Tyr Tyr Pro Thr Glu Val Trp Tyr
       50                  55                  60
   Asn Cys Ser Arg Ser Ala Thr Thr Thr Ala Tyr Lys Asp Phe Ser Asn
   65                  70                  75                  80
   Ile His Ala Phe Tyr Phe Asp Met Glu Ala Met Glu Asn Ser Thr Gly
                   85                  90                  95
   Asn Ala Arg Gly Lys Pro Leu Leu Val His Val His Gly Asp Pro Val
               100                 105                 110
   Ser Ile Ile Ile Tyr Ile Ser Ala Tyr Arg Asp Asp Val Gln Gly Arg
           115                 120                 125
   Pro Leu Leu Lys His Gly Leu Leu Cys Ile Thr Lys Asn Lys Ile Ile
       130                 135                 140
   Asp Tyr Asn Thr Phe Thr Ser Ala Gln Trp Ser Ala Ile Cys Leu Gly
   145                 150                 155                 160
   Asp Asp Arg Lys Ile Pro Phe Ser Val Ile Pro Thr Gly Asn Gly Thr
                   165                 170                 175
   Lys Ile Phe Gly Leu Glu Trp Asn Asp Asp Tyr Val Thr Ala Tyr Ile
               180                 185                 190
   Ser Asp Arg Ser His His Leu Asn Ile Asn Asn Asn Trp Phe Asn Asn
           195                 200                 205
   Val Thr Ile Leu Tyr Ser Arg Ser Ser Thr Ala Thr Trp Gln Lys Ser
       210                 215                 220
   Ala Ala Tyr Val Tyr Gln Gly Val Ser Asn Phe Thr Tyr Tyr Lys Leu
   225                 230                 235                 240
   Asn Asn Thr Asn Gly Leu Lys Ser Tyr Glu
                   245                 250
(2) INFORMATION FOR SEQ ID NO:58:
   (i) SEQUENCE CHARACTERISTICS:
      (A) LENGTH: 201 amino acids
      (B) TYPE: amino acid
      (D) TOPOLOGY: unknown
   (ii) MOLECULE TYPE: protein
   (xi) SEQUENCE DESCRIPTION: SEQ ID NO:58:
   Ser Phe Asn Leu Thr Thr Gly Asp Ser Gly Ala Phe Trp Thr Ile Ala
   1               5                   10                  15
   Tyr Thr Ser Tyr Thr Asp Ala Leu Val Gln Val Glu Asn Thr Ala Ile
               20                  25                  30
   Lys Lys Val Thr Tyr Cys Asn Ser His Ile Asn Asn Ile Lys Cys Ser
           35                  40                  45
   Gln Leu Thr Ala Asn Leu Gln Asn Gly Phe Tyr Pro Val Ala Ser Ser
       50                  55                  60
   Glu Val Gly Leu Val Asn Lys Ser Val Val Leu Leu Pro Ser Phe Tyr
   65                  70                  75                  80
   Ser His Thr Ser Val Asn Ile Thr Ile Asp Leu Gly Met Lys Arg Ser
                   85                  90                  95
   Gly Tyr Gly Gln Pro Ile Ala Ser Thr Leu Ser Asn Ile Thr Leu Pro
               100                 105                 110
   Met Gln Asp Asn Asn Thr Asp Val Tyr Cys Ile Arg Ser Asn Gln Phe
           115                 120                 125
   Ser Val Tyr Val His Ser Thr Cys Lys Ser Ser Leu Trp Asp Asp Val
       130                 135                 140
   Phe Asn Ser Asp Cys Thr Asp Val Leu Tyr Ala Thr Ala Val Ile Lys
   145                 150                 155                 160
   Thr Gly Thr Cys Pro Phe Ser Phe Asp Lys Leu Asn Asn Tyr Leu Thr
                   165                 170                 175
   Phe Asn Lys Phe Cys Leu Ser Leu Asn Pro Val Gly Ala Asn Cys Lys
               180                 185                 190
   Phe Asp Val Ala Ala Arg Thr Arg Thr
           195                 200
(2) INFORMATION FOR SEQ ID NO:59:
   (i) SEQUENCE CHARACTERISTICS:
      (A) LENGTH: 251 amino acids
      (B) TYPE: amino acid
      (D) TOPOLOGY: unknown
   (ii) MOLECULE TYPE: protein
   (xi) SEQUENCE DESCRIPTION: SEQ ID NO:59:
   Glu Asn Met Glu Ile Asp Ser Met Leu Phe Val Ser Glu Asn Ala Leu
   1               5                   10                  15
   Lys Leu Ala Ser Val Glu Ala Phe Asn Ser Thr Glu Thr Leu Asp Pro
               20                  25                  30
   Ile Tyr Lys Glu Trp Pro Asn Ile Gly Gly Ser Trp Leu Gly Gly Leu
           35                  40                  45
   Lys Asp Ile Leu Pro Ser His Asn Ser Lys Arg Lys Tyr Arg Ser Ala
       50                  55                  60
   Ile Glu Asp Leu Leu Phe Asp Lys Val Val Thr Ser Gly Leu Gly Thr
   65                  70                  75                  80
   Val Asp Glu Asp Tyr Lys Arg Cys Thr Gly Gly Tyr Asp Ile Ala Asp
                   85                  90                  95
   Leu Val Cys Ala Gln Tyr Tyr Asn Gly Ile Met Val Leu Pro Gly Val
               100                 105                 110
   Ala Asn Asp Asp Lys Met Ala Met Tyr Thr Ala Ser Leu Ala Gly Gly
           115                 120                 125
   Ile Thr Leu Gly Ala Leu Gly Gly Gly Ala Val Ser Ile Pro Phe Ala
       130                 135                 140
   Ile Ala Val Gln Ala Arg Leu Asn Tyr Val Ala Leu Gln Thr Asp Val
   145                 150                 155                 160
   Leu Ser Lys Asn Gln Gln Ile Leu Ala Asn Ala Phe Asn Gln Ala Ile
                   165                 170                 175
   Gly Asn Ile Thr Gln Ala Phe Gly Lys Val Asn Asp Ala Ile His Gln
               180                 185                 190
   Thr Ser Gln Gly Leu Ala Thr Val Ala Lys Ala Leu Ala Lys Val Gln
           195                 200                 205
   Asp Val Val Asn Thr Gln Gly Gln Ala Leu Ser His Leu Thr Val Gln
       210                 215                 220
   Leu Gln Asn Asn Phe Gln Ala Ile Ser Ser Ser Ile Ser Asp Ile Tyr
   225                 230                 235                 240
   Asn Arg Leu Asp Glu Leu Ser Ala Asp Ala Gln
                   245                 250